(19) Europäisches Patentamt

European Patent Office

Office européen des brevets

(11) **EP 1 626 274 A1**

(12) **EUROPEAN PATENT APPLICATION**
published in accordance with Art. 158(3) EPC

(43) Date of publication:
15.02.2006 Bulletin 2006/07

(51) Int Cl.:
*G01N 27/62* (1968.09)  *G01N 30/72* (1985.01)

(21) Application number: 04724777.0

(22) Date of filing: 31.03.2004

(86) International application number:
PCT/JP2004/004621

(87) International publication number:
WO 2004/090526 (21.10.2004 Gazette 2004/43)

(84) Designated Contracting States:
CH DE DK GB LI SE

(30) Priority: 31.03.2003 JP 2003095732

(71) Applicant: Medical Proteoscope Co., Ltd.
Shinjuku-ku,
Tokyo 1630207 (JP)

(72) Inventors:
• OGIWARA, Atsushi
Hachioji-shi,
Tokyo 1930942 (JP)

• KAWAKAMI, Takao,
No, 303 Yoshino Manshon, 4-19-2
Saitama 3360022 (JP)
• NISHIMURA, Toshihide
3001243 (JP)

(74) Representative: Denison, Christopher Marcus et al
Mewburn Ellis LLP
York House
23 Kingsway
London WC2B 6HP (GB)

(54) **SAMPLE ANALYZING METHOD AND SAMPLE ANALYZING PROGRAM**

(57) A sample analyzing method and a sample analyzing program for analyzing components contained in a sample with excellent analysis ability. The sample analyzing method of the present invention comprises: a step (a) of correcting at least a one-dimensional parameter in multi-dimensional data obtained as a result of the analysis of a sample; and a step (b) of comparing the corrected data obtained in said step (a) for multiple samples.

Fig. 1

EP 1 626 274 A1

SEQUENCE LISTING

<110> Medical Proteoscope Co., Ltd

<120> Sample Analyzing Method and Sample Analyzing Program

<130> CMD/FP6333173

<140> EP 04724777.0
<141> 2004-03-31

<150> PCT/JP2004/004621
<151> 2004-03-31

<150> JP 2003-095732
<151> 2003-03-31

<160> 2

<170> PatentIn version 3.1

<210> 1
<211> 7
<212> PRT
<213> Artificial sequence

<220>
<221> source
<223> /note = "Description of artificial sequence:   Peptide T, used as a
            reference material"

<400> 1

Ala Ser Thr Thr Asn Tyr Thr
1               5

```
<210>  2
<211>  7
<212>  PRT
<213>  Artificial sequence

<220>
<221> source
<223> /note = "Description of artificial sequence:   beta-casomorphin-7,
             used as a reference material"

<400>  2


Tyr Pro Phe Pro Gly Pro Ile
1               5
```

**Description**

Technical Field

The present invention relates to a sample analyzing method and a sample analyzing program using multi-dimensional data obtained as a result of the analysis of a sample.

Background Art

For example, as a result of liquid chromatography mass spectrometry (hereinafter abbreviated as LC-MS) in which liquid chromatography (hereinafter abbreviated as LC) is combined with mass spectrometry (hereinafter abbreviated as MS), spectrum data can be obtained on a two-dimensional graph, in which the horizontal axis is defined as a mass-to-charge ratio (hereinafter abbreviated as m/z) and the longitudinal axis is defined as ionic intensity. Herein, the role of LC is to simply fractionate a sample, so as to adapt it to the processing ability of MS.

That is to say, the aforementioned two-dimensional spectrum data can be obtained by analyzing a sample fractionated by LC according to MS, thereby analyzing components contained in the sample. However, the conventional LC-MS has been problematic in that since the role of LC is simply limited to fractionation, the types of proteins that can be detected or identified in an analyte are not comprehensive, and thus that its analytical capacity is low.

For the purpose of utilizing chromatography not only for fractionation, but also as information showing the properties of a sample, several methods of correcting and aligning time bases to compare the results of multiple chromatographs have been proposed. Typical examples of such a method may include Dynamic Time Warping (hereinafter abbreviated as DTW) and Correlation Optimized Warping (hereinafter abbreviated as COW). In both methods, Euclidean distance or correlation is used as an index of the distance or similarity of two chromatograms as an implementation form based on dynamic programming algorithm (V. Pravdova, B. Walczak, D. L. Massart, "A comparison of two algorithms for warping of analytical signals," Anal. Chim. Acta 456: 77-92 (2002)). However, since these methods are applied to a chromatogram expressed in two dimensions, a time base and signal intensity in chromatography, they do not intend to correct at least a one-dimensional parameter in multi-dimensional data.

Moreover, such an alignment method is predicated upon the condition where chromatograms or spectrograms as comparison targets are similar to each other to a certain extent. In reality, in both DTW and COW, since such alignment is carried out for the minimization of a distance between profiles as comparison targets or the maximization of a correlation thereof, it is highly likely that an appropriate alignment cannot be achieved when such profiles as comparison targets have a low commonality. Thus, it is inappropriate to apply such methods premised on a high commonality to the analysis of practical diseases or pathologic conditions or the analysis of drug response, in which fluctuation in many factors is anticipated, and further in which the amount of such fluctuation is very small and thus the fluctuation is mixed in individual difference or measurement error.

The present invention has been completed under the aforementioned present circumstances. Thus, it is an object of the present invention to provide a sample analyzing method and a sample analyzing program, which exert excellent analysis ability when components contained in a sample is analyzed.

Disclosure of the Invention

The present invention that has achieved the aforementioned object includes the following features:

(1) a sample analyzing method, which comprises: a step (a) of correcting at least a one-dimensional parameter in multi-dimensional data obtained as a result of the analysis of a sample; and a step (b) of comparing the corrected data obtained in the above-described step (a) for multiple samples.

In the present sample analyzing method, the above-described multi-dimensional data may be three-dimensional data consisting of a parameter indicating a mass-to-charge ratio, a parameter indicating ionic intensity, and a parameter indicating a retention time, the parameters obtained as a result of chromatography mass spectrometry. In addition, the parameter indicating a retention time is preferably corrected in the above-described step (a).

Moreover, herein, profiles regarding parameters, from which a parameter as a correction target has been excluded, are defined as reference profiles, and an evaluation function acting as a scale of position similarity regarding a plurality of reference profiles among multiple samples is given. In this case, the position of each profile can be determined as a problem of finding an optimum solution which optimizes the value of the above-described evaluation function in the above-described step (a).

Herein, the above-described evaluation function is preferably defined with one or more terms selected from the group consisting of the following terms (1) to (5):

(1) a term regarding similarity and/or distance among profiles regarding a parameter of a correction target;

(2) a term regarding similarity and/or distance among profiles regarding a reference profile;

(3) a term regarding the degree of concordance of data points among profiles as comparison targets;

(4) a term regarding the degree of discordance of data points among profiles as comparison targets;

(5) a term regarding the degree of concordance or discordance of reference material-derived signals among profiles as comparison targets; and

(6) a term regarding the degree of concordance in the previous comparison during repeated comparison operations.

Furthermore, in the above-described step (a), dynamic programming algorithm can be used, when the value of the above-described evaluation function is optimized as a problem of finding an optimum solution regarding the above-described parameter of a correction target. In the above-described dynamic programming algorithm, when the optimal correspondence of data points contained in a parameter of a correction target is evaluated by calculating scores, the score of a correspondence regarding data points derived from a reference material is preferably set by a point-addition scoring system. Further, in this case, a constraint condition, in which a correspondence regarding data points derived from a reference material is necessarily corresponded at a designated point, is preferably set.

In the sample analyzing method described in (1) above, using information derived from a reference material that had previously been added, in particular, in the above-described step (a), the analytical precision can be improved, and correction processing ability can also be improved. Among the sample analyzing methods of the present invention, a method with such characteristics is named as an internal standard guided optimal profile alignment (i-OPAL) method.

Moreover, the aforementioned sample analyzing method of the present invention can be realized in the form of a program for allowing a computer to execute, in which the computer comprising an input means for inputting various types of data, an processing means for performing operations in accordance with the program, and a display means for displaying the results of the above operations or the like.

Also, the sample analyzing method of the present invention enables detection and/or identification of substances with different amounts in different types of samples. Specifically, three-dimensional data consisting of a parameter indicating a mass-to-charge ratio, a parameter indicating ionic intensity, and a parameter indicating a retention time, obtained as a result of chromatography mass spectrometry, are measured as multi-dimensional data in multiple samples. Such three-dimensional data are compared for multiple samples, and signals with significantly different ionic intensity can be detected and/or identified. A substance generating signals having the properties of which are sufficiently similar to the properties of the above signals, such as a mass-to-charge ratio and a retention time, is further analyzed, so as to identify the above substance.

Furthermore, such detection and/or identification steps are applied to a sample derived from disease and a sample derived from a healthy subject or healthy tissues, so as to detect and/or identify a substance showing the abundance of which is significantly different between such a disease group and a healthy group. The thus identified substance can be used as a biomarker. The results of detection and/or identification of such a biomarker can be used for the diagnosis of diseases or selection of a therapeutic method.

Still further, such detection and/or identification steps are applied to samples derived from a group of patients who exhibit different response or have different side effects to a specific therapeutic method or a drug, so that the steps can be used for detection and/or identification of a substance acting as a marker of a therapeutic method/drug response or side effects thereby.

This specification includes part or all of the contents as disclosed in the specification and/or drawings of Japanese Patent Application No. 2003-95732, which is a priority document of the present application.

Brief Description of the Drawings

Figure 1 is a view showing an example of three-dimensional spectrum data obtained by the sample analyzing method and sample analyzing program of the present invention;

Figure 2 is a view showing an example of three-dimensional data;

Figure 3 is a view showing an example of another three-dimensional data that are established to search for a correspondence with the three-dimensional data shown in Figure 2;

Figure 4 is a view showing the optimum correspondence position of the three-dimensional data shown in Figure 2 with the three-dimensional data shown in Figure 3;

Figure 5 is a view showing the concept regarding a search for the optimum correspondence position of the three-dimensional data shown in Figure 2 with the three-dimensional data shown in Figure 3;

Figure 6 is a view showing that if the pathway is limited using information derived from a reference material in the searching for the optimum position shown in Figure 5, the grey portion in the space to be searched does not need to be searched any more;

Figure 7 is a view showing that if information derived from a reference material is more often used so as to increase the pathway constraint conditions, a space that does not need to be searched increases, and searching efficiency is thereby further improved;

Figure 8 is a view showing the results obtained by aligning waveforms that surge on the time base in 5 measurement results of a single type, and adding signals, according to the sample analyzing program of the present invention;

Figure 9 is a view showing that samples at different time points can be compared with each other by aligning profiles obtained by the measurement of 7 different samples based on the same time base, according to the sample analyzing program of the present invention;

Figure 10 is a view showing a difference spectrum between two different types of samples that is calculated according to the sample analyzing program of the present invention;

Figure 11 is a view showing signals that are selected as those with significant differences in amounts among sample groups according the sample analyzing program of the present invention;

Figure 12 is a view showing that the sample analyzing program of the present invention is applied to search for a marker using actual clinical analytes, so that signals can be classified depending on grouping due to different pathologic diagnoses;

Figure 13 is a view showing that a further statistical test is conducted on the results shown in Figure 12, so as to pick out signals that quantitatively change depending on different pathologic diagnoses;

Figure 14 is a view showing the results obtained by further correlating each signal in the results shown in Figure 13 with identification of a protein by MS/MS; and

Figure 15 is a table showing a part of the results of proteins that have known to be particularly associated with cancer metastasis, which are obtained according to the sample analyzing system of the present invention, from among the signals that have been correlated with known proteins in the form that is shown in Figure 14.

Best Mode for Carrying Out the Invention

The present invention will be described in detail below with reference to the drawings.

1. Preparation of sample

In the sample analyzing method of the present invention, a sample as an analysis target is first collected. The type of a sample to be analyzed is not particularly limited. Examples of a sample may include: tissue section of organs derived from individual animals; body fluid components such as plasma or lymth; plant organs such as green leaves or petal; and soil or water components existing in the environment. The type of a substance as an analysis target contained in these samples is not particularly limited. Examples of such a substance may include an organic compound, an inorganic compound, an organic metal compound, a metal ion, a peptide, a protein, a metalloprotein, a peptide that has been subjected to post-translational modification including phosphorylation, a protein that has been subjected to post-translational modification including phosphorylation, a nucleic acid, a carbohydrate, and a lipid. Particularly preferred examples may include a peptide, a protein, a metalloprotein, and a peptide and a protein that have been subjected to post-translational modification.

The collected sample may preferably be subjected to various types of treatments, as necessary, depending on the purpose of analysis and the properties of the collected samples. For example, it is preferable to perform a preparation before analysis, which includes all of the following elements (1) to (4), or several elements thereof in combination: (1) separation or fractionation of a group of proteins; (2) enzymatic and/or chemical cleavage of a group of proteins; (3) separation or fractionation of a peptide mixture generated as a result of a cleavage; and (4) addition of a reference material.

More specifically, such "(1) separation or fractionation of a group of proteins" can be carried out by one-dimensional sodium dodecyl sulfate (SDS) electrophoresis, two-dimensional electrophoresis, capillary electrophoresis, ion exchange chromatography, gel filtration chromatography, normal phase chromatography, reverse phase chromatography, affinity chromatography, or multi-dimensional separation and/or fractionation performed by the combined use of the above methods.

In addition, "(2) enzymatic and/or chemical cleavage of a group of proteins" can be carried out by trypsin digestion, chymotrypsin digestion, Lys-C digestion, Asp-N digestion, Glu-C digestion, cyanogen bromide decomposition, cleavage by the combination thereof, or the like.

Moreover, "(3) separation or fractionation of a peptide mixture generated as a result of the cleavage" can be carried out by one-dimensional sodium dodecyl sulfate (SDS) electrophoresis, two-dimensional electrophoresis, capillary electrophoresis, ion exchange chromatography, gel filtration chromatography, normal phase chromatography, reverse phase chromatography, affinity chromatography, or multi-dimensional separation and/or fractionation performed by the combined use of the above methods.

Furthermore, as a reference material used in "(4) addition of a reference material," it is preferable to select a material, which can be ionized by the selected ionization method, is eluted within a range of the LC retention time of measurement, and has high reproducibility in elution time and molecular ion intensity. Examples of such a preferred reference material may include an organic compound, an inorganic compound, an organic metal compound, a metal ion, a peptide, a protein, a metalloprotein, a peptide that has been subjected to post-translational modification including phosphorylation, a protein that has been subjected to post-translational modification including phosphorylation, a nucleic acid, a carbohydrate, and a lipid. More preferably, a commercially available peptide or protein product, a naturally existing substance, and a synthesized substance can be used.

The aforementioned various types of treatments (1) to (4) can be carried out in the order of "(1), (4), (2), and (3)," "(4), (2), and (3)," "(2) (4), and (3)," "(4) and (1)," "(4) and (2)," "(2) and (4)," or only (4)."

2. Sample analysis

Subsequently, a sample is analyzed so as to obtain multi-dimensional data regarding the sample. Specifically, a sample is analyzed by LC-MS, so as to measure multi-dimensional data consisting of m/z, ionic intensity, and retention time. Herein, analysis by LC-MS means that a sample is separated or fractionated in accordance with the principle of chromatography, and that components contained in the separated or fractionated sample are then measured in accordance with the principle of mass spectrometry. In addition, retention time means a period of time required for separation or fractionation of a sample in accordance with the principle of chromatography. Moreover, m/z and ionic intensity are measured, as the results obtained by the measurement of mass spectrometry.

Furthermore, such principle of chromatography is not particularly limited. Various types of principles of chromatography can be applied. Examples may include reverse phase chromatography, capillary electrophoresis, affinity chromatography, chromatofocusing, isoelectric focusing, and gel filtration chromatography. In particular, when the symbol LC is used in the present specification, it means not only liquid chromatography, but also general chromatography with a broad sense.

Chromatography in LC-MS is preferable in that an elution profile with high reproducibility can be obtained, in that it has high separation ability, and in that molecular ions can directly be introduced into MS via an appropriately ionized interface.

More specifically, preferred conditions in liquid chromatography are as follows. When a group of peptides in a sample is used as an analysis target, it is preferable to apply reverse phase liquid chromatography with a C18 column, using an eluent produced by adding strong acid such as formic acid with a low concentration to water or an acetonitrile solution. On the other hand, when a group of proteins in a sample is used as an analysis target, it is preferable to apply reverse phase liquid chromatography with a C4 column, using an eluent produced by adding strong acid such as formic acid with a low concentration to water or an acetonitrile solution.

The type of mass spectrometry is not particularly limited. Examples of a mass spectrometer used herein may include a magnetic field mass spectrometer, a time of flight mass spectrometer, a quadrupole mass spectrometer, an ion trap mass spectrometer, a Fourier transform mass spectrometer, and a hybrid mass spectrometer thereof and a tandem mass spectrometer thereof. More preferably, a magnetic field mass spectrometer, a time of flight mass spectrometer, a quadrupole mass spectrometer, an ion trap mass spectrometer, a Fourier transform mass spectrometer, a hybrid mass spectrometer thereof, or a tandem mass spectrometer thereof, which can be used in combination with electrospray ionization or nanoelectrospray ionization, is preferably used to carry out mass spectrometry.

Mass spectrometry in LC-MS is preferable in that a mass spectrum with high reproducibility can be obtained, in that it has a high mass precision of 500 ppm or less, and in that a mass spectrum of fragment ions of molecular ions can be obtained by multiplying the molecular ions within a certain range of m/z by collision induced dissociation (CID).

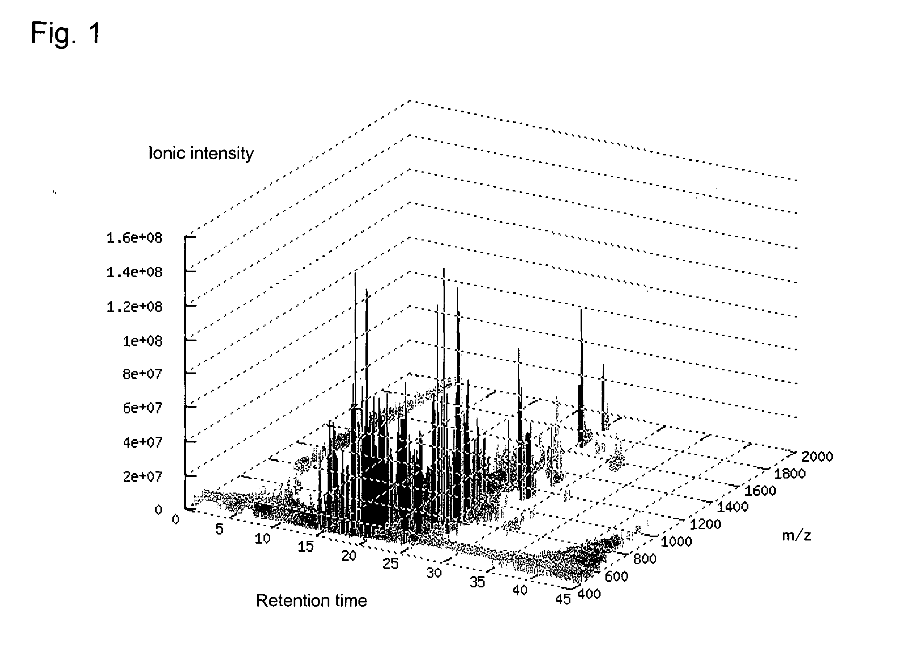

Thus, a sample is analyzed by LC-MS, so as to measure m/z, ionic intensity, and retention time, thereby obtaining the analysis results of the sample in the form of three-dimensional data. In the LC-MS analysis, data regarding retention time, signals regarding m/z, and data regarding ionic intensity, are inputted in a computer via an input means, and thereafter, such data are processed by an processing means in accordance with algorithm, the detail of which will be described later, so as to obtain three-dimensional data as shown in Figure 1. This algorithm can be incorporated into computer software. Thereafter, the software is installed into a computer, so that the algorithm can be realized on a computer via an processing means such as CPU. Accordingly, three-dimensional data as shown in Figure 1 can be displayed on a display device of the computer.

In the conventional LC-MS analysis method, LC has been carried out only for fractionation of a sample. Thus, retention time has not been used as a parameter of an analysis target, and two-dimensional data (horizontal axis: m/z; and longitudinal axis: ionic intensity) obtained as analysis results of a sample have only been used as analysis targets. In contrast, according to the analysis method of the present invention, the analysis results of a sample can be obtained in the form of a profile in which the results are plotted on a three-dimensional space, and thus the ability to analyze a sample can dramatically be improved. More specifically, according to the analysis method of the present invention, data can be obtained in the form of a large number of spectra that are aligned and broaden in the direction of an axis indicating retention time. Thus, when compared with the conventional analysis method, a larger number of components can be identified based on such spectra. Accordingly, for example, the obtained multi-dimensional data to multiple samples are compared, so as to carry out the component analysis of each sample in a more strict manner.

3. Data analysis

Subsequently, in the analysis method of the present invention, the retention time that is measured as described above can be corrected by the algorithm of the present invention under the control of a processing means. In general, retention time nonlinearly fluctuates in many cases, since factors such as the composition of a mobile phase, a flow rate, or a column temperature in LC cause a minute change in time. Accordingly, it is considered that, in the case of three-dimensional data obtained by the analysis method of the present invention as well, when multiple samples are analyzed, an axis indicating retention time may nonlinearly fluctuate among samples. Hence, in the algorithm of the present invention, correction of such retention time (hereinafter referred to also as "time base correction" at times) is carried out.

However, such "time base correction" conducted in the algorithm of the present invention is not the same as the correction of a single dimensional profile represented by a two-dimensional space consisting of retention time and signal intensity, such as time base correction in a chromatogram according to the conventional method using DTW algorithm or the like. In data targeted by the present invention, a profile to be corrected regarding time base is represented by a multi-dimension, at least a two-dimension.

The present algorithm will be described below. The application of the present algorithm is not limited to correction of retention time, but it can widely be applied to a case where at least a one-dimensional parameter is corrected when multi-dimensional parameters are obtained. In other words, the present algorithm can be applied, when at least a one-dimensional parameter is corrected among multi-dimensional parameters (for example, three-dimensional parameters) obtained as a result of the analysis of a sample. Accordingly, an algorithm in a case where a $p + q$ dimensional measurement data have been obtained will be described below.

First, when the measurement value of $p$ dimensions including a parameter as a correction target is indicated as $(\mathbf{x_1} ... \mathbf{x_p})$ and the measurement value of q dimensions that is referred during the correction is indicated as $(\mathbf{y_1} ... \mathbf{y_q})$, an aggregation of data (profile) z is indicated as $\mathbf{z} = (\mathbf{x_1} ... \mathbf{x_p} \ \mathbf{y_1} ... \mathbf{y_q})$. Herein, $\mathbf{x}$ and $\mathbf{y}$ are column vectors having a dimension of an N number of data points.

A data point means a vector with $p + q$ dimensions that constitutes a single row of the aforementioned profile matrix (z). It represents a set of parameters consisting of measurement parameters and values for one instance of the measurement target. In particular, a data point at position $n \in \{1,...,N\}$ may be represented by

$$\overline{Z(n)} = \begin{pmatrix} x_1(n) & \cdots & x_p(n) & y_1(n) & \cdots & y_q(n) \end{pmatrix}.$$

In addition, the measurement value as a reference of correction is represented by

$$\overline{Z(*s)} = \begin{pmatrix} x_1(*s) & \cdots & x_p(*s) & y_1(*s) & \cdots & y_q(*s) \end{pmatrix}.$$ Herein, $s$ means 1 to $S$ (wherein $S$ represents the number of reference points). Moreover, in $\overline{Z(*s)}$, all the values of reference points should be within a range that can be estimated.

Furthermore, in order to conduct correction in the present algorithm, two or more profile data,

$$\mathbf{Z^{(1)}} = \begin{pmatrix} \mathbf{x_1^{(1)}} & \cdots & \mathbf{x_p^{(1)}} & \mathbf{y_1^{(1)}} & \cdots & \mathbf{y_q^{(1)}} \end{pmatrix} \text{ and } \mathbf{Z^{(2)}} = \begin{pmatrix} \mathbf{x_1^{(2)}} & \cdots & \mathbf{x_p^{(2)}} & \mathbf{y_1^{(2)}} & \cdots & \mathbf{y_q^{(2)}} \end{pmatrix} \text{ are required.}$$

Under the aforementioned definition, the values that can be obtained in a $p$ number of parameter axes $x_l \cdots x_p$ are first quantized in the present algorithm. However, such quantization process is carried out in balance of calculation precision and calculation time. Thus, if the value is within a range that can sufficiently be calculated, the quantization process is not necessarily carried out at this stage. Subsequently, the data points of $\mathbf{x_i^{(1)}}$ and $\mathbf{x_i^{(2)}}$ (wherein $i \in \{1,..., p\}$) are allowed to correlate to each other, while keeping the permutation, in each of the $p$ number of parameter axes $x_1$

··· $x_p$. In general, since the number of data points included in $\mathbf{Z}^{(1)}$ and $\mathbf{Z}^{(2)}$ may be different, it should be noted that all data points are not correlated to one by one, but that data points that have no corresponding parties may also included.

At this stage, an evaluation score E regarding correlation in the profile as a whole is calculated, using an evaluation function described below, for example. This evaluation score can be defined as a "gain" that is a scale representing similarity. In this case, the larger the value of the "gain", the better the score that can be obtained. Otherwise, the evaluation score can also be defined as a "loss" that is a scale representing distance. In this case, the smaller the value of the "loss", the better the score that can be obtained. Hereafter, the definition as a loss will be described.

$$E = \sum_{i=1, j=1}^{N_1, N_2} f\left(x_1^i, x_1^j, \ldots, x_p^i, x_p^j, y_1^i, y_1^j, \ldots, y_q^i, y_q^j\right)$$

Herein, $x_r^i$ represents the value of the rth parameter in the ith data point, and $N_1$ and $N_2$ represent the total number of data points at the first and second profiles, respectively. In addition, in the aforementioned evaluation function, the function $f$ is a function that gives the distance of the similarity degree at a corresponding point. The following function may be an example of such function $f$:

$$f\left(x_1^i, x_1^j, \ldots, x_p^i, x_p^j, y_1^i, y_1^j, \ldots y_q^i, y_q^j\right)$$
$$= \sum_{r=1}^{p} \alpha_r \cdot \delta_r(i, j) \cdot \left\| x_r^i - x_r^j \right\| + \sum_{s=1}^{q} \beta_s \cdot \left\| y_s^i - y_s^j \right\| - \sigma \prod_{r=1}^{p} \delta_r(i, j) + \sum_{r=1}^{p} \pi_r \cdot \overline{\delta_r(i, j)} - \theta(i, j) \qquad \ldots \text{(I)}$$

Herein, in the aforementioned formula (I), the first item on the right-hand side represents a penalty that depends on the degree of difference in the measure of a parameter $x_r$ to be corrected; the second item represents a penalty that depends on the distance measured along the parameter, which indicates the level of deviance regarding a measurement parameter $y_s$ to be aligned that has deviated after correction; the third item represents a score that is given as a bonus for the result that two points are matched at all parameters as a result of parameter correction; and in contrast, the fourth item represents a penalty score that is given for the result that the two points are not matched on the axis of a parameter as a correction target. Further, the fifth item is for evaluating the concordance of signals due to a reference material as a bonus, as described below.

Moreover, α, β, σ, and π in the aforementioned formula (I) are coefficients in the items including each of these. These values can be determined as appropriate. As an example, α is defined as 1.0, and β is defined as 0.1. When points are matched by parameter correction, σ is defined as 0. When these points are not matched, π is defined as 100.

The function $\delta_r(i, j)$ gives 1, when the value of the parameter $r$ to be focused corresponds at a data point designated by $i$ and $j$. The above function gives 0, when the above value does not correspond at the above point. In contrast, the function $\overline{\delta_r(i, j)}$ gives 0 when it corresponds, and gives 1 when it does not correspond.

In the aforementioned formula (I), the second item represents a scale of position similarity between samples, with regard to a profile regarding parameters from which a parameter as a correction target has been excluded (a reference profile).

Herein, an example is given, in which a constant can be obtained depending on correspondence or non-correspondence, using a formula for giving a penalty due to discordance of two points. However, a value that can be calculated by a certain function may also be applied. For example, the fourth item can be calculated using a function, in which whether or not the adjacent data point corresponds or the length of a column in which non-corresponding data point appears are taken in consideration.

Moreover, in the aforementioned formula (I), the norm $\|x\|$ represents a distance on a common vector space, and it is not necessarily limited to the Euclidean distance. Furthermore, in a case where no corresponding points exist when the difference between the values of two points is calculated using $\left\| y_s^i - y_s^j \right\|$, , for example, the calculation is carried out by replacing the value with 0 (or an appropriate alternative value for an absence value).

Furthermore, the evaluation function in the present invention is not limited to the function represented by the aforementioned formula (I). For example, any given function, which involves not only the linear combination of the distance of a correction target parameter or reference parameter between such data points ($i, j$) but also the distance between them, and further, a function, which involves the distance between parameters in a column of data points immediately before or that have continuously corresponded until that time, can also be used as evaluation functions in the present invention. Further, such an evaluation functions is not limited to that represented by the aforementioned formula (I), but a function acting as a scale of position similarity of a reference profile between samples can also be used.

An example of a loss is given herein. However, it is also easily possible to define an evaluation function as an index of similarity by inverting the signs in each item on the right-hand side of the aforementioned formula (I) and by substituting the distance portion with correlation or the like. In such a function, the larger the value of the "gain", the better the score that can be obtained. Such an evaluation function can also be applied to the present algorithm.

As shown in the fifth item of the aforementioned formula (I), the following special score can be given for example, depending on whether or not the corresponding point is a reference point derived from a reference material. That is to say, when all the corresponding data points are derived from a reference material, a much greater score can be set by the formula $\theta(i, j) = S_m$, so that the evaluation function (in this case, defined as a distance, that is, a loss) can give a large negative value. As a result, it is defined that such a corresponding relationship is preferable. On the other hand, when one corresponding data point is derived from a reference material but the other is not, on the contrary, it is also possible to define such that a much greater distance can be set by the formula $\theta(i, j) = -S_d$.

Using the above-described algorithm for optimizing an evaluation function, with regard to the three-dimensional data obtained in the above section "2. Sample analysis," a parameter indicating retention time can be corrected. When the optimization algorithm is applied to the three-dimensional data obtained in the above section "2. Sample analysis," the procedures are described in following (a) to (d).

(a) Concept of correction of retention time

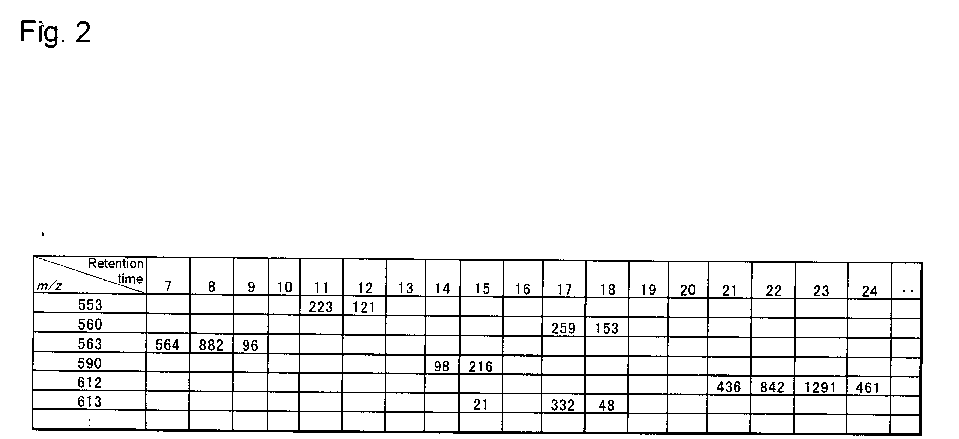

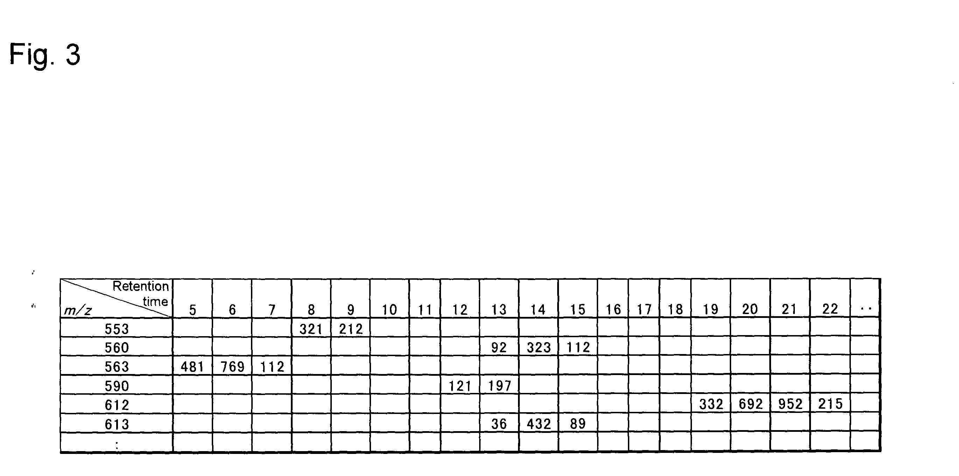

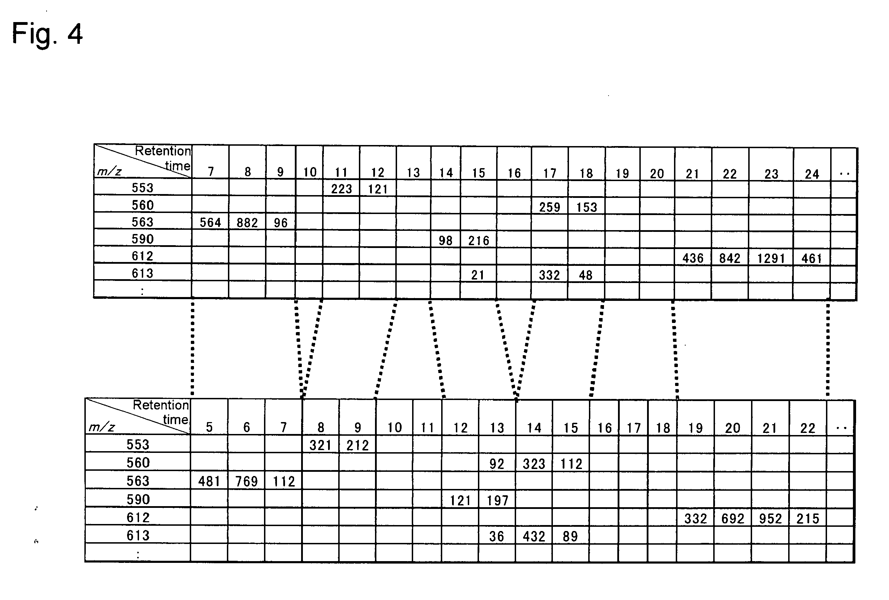

The operation to correct retention time is not targeted to a single three-dimensional parameter aggregate consisting of m/z, ionic intensity, and retention time, but it is realized by comparison between two three-dimensional parameter aggregates. As shown in Figure 2, a three-dimensional parameter aggregate is expressed in a matrix wherein m/z and retention time are defined as a row and a column, respectively, and wherein ionic intensity is included in the matrix element at the position to which m/z and retention time correspond. When three-dimensional parameter aggregates, the retention time of which is to be corrected, are defined as $Z^{(1)}$ and $Z^{(2)}$, the operation to correct the retention time is nothing but the operation to determine the corresponding relationship of a column corresponding to the retention time axis in two matrixes in $Z^{(1)}$ and $Z^{(2)}$ (hereinafter referred to as a "search for corresponding position"). For example, when the matrix shown in Figure 2 is defined as matrix $Z^{(1)}$ and the matrix shown in Figure 3 is defined as matrix $Z^{(2)}$, the position shown in Figure 4 is a preferred corresponding position (aligned position).

(b) Search for corresponding position between parameter aggregates in two three-dimensional data

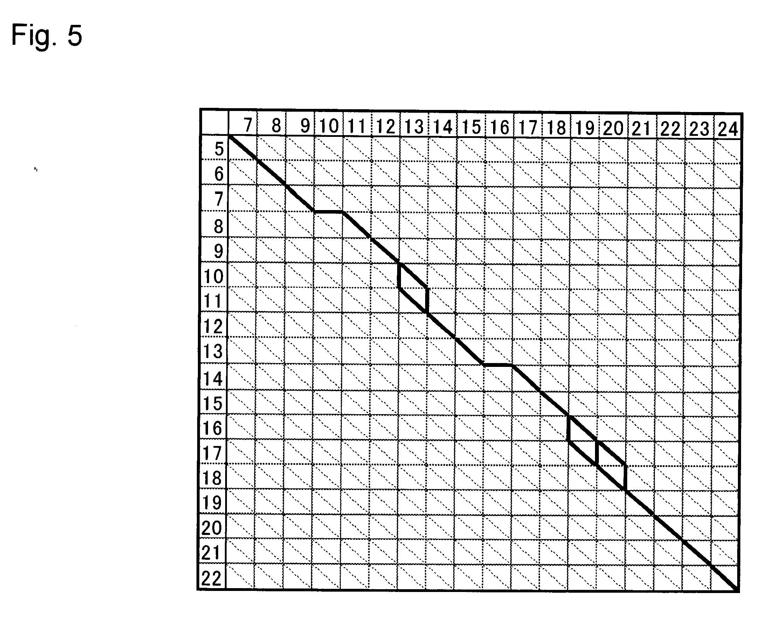

In order to search for corresponding position shown in Figure 4, all possible correspondences of retention time are conceived. Herein, a score is defined to evaluate the correspondence of position, and such a score is calculated for every position. Among the calculated scores, the best score is adopted, so as to obtain the optimum corresponding position of interest. Figure 5 shows all possible correspondences of retention time for the three-dimensional parameter aggregates, $Z^{(1)}$ and $Z^{(2)}$, shown in Figures 2 and 3. The horizontal direction represents the retention time of $Z^{(1)}$, and the longitudinal direction represents the retention time of $Z^{(2)}$. When both $Z^{(1)}$ and $Z^{(2)}$ have the corresponding retention times, it is indicated with an diagonal line (case (1)). When $Z^{(2)}$ does not have a retention time corresponding to the certain retention time of $Z^{(1)}$, it is indicated with a horizontal line (case (2)). When $Z^{(1)}$ does not have a retention time corresponding to the certain retention time of $Z^{(2)}$, it is indicated with a longitudinal line (case (3)). The correspondence of the total retention times of $Z^{(1)}$ and $Z^{(2)}$ can be obtained by tracing these diagonal, horizontal, and longitudinal lines along the pathway from the upper leftmost angle to the lower rightmost angle in the lattices shown in Figure 5. However, once the pathway goes down or proceeds to the right, it is not allowable for the pathway to return to the position upward or to the left. The pathway indicated with a bold line in Figure 5 corresponds to the corresponding position shown in Figure 4.

(c) Score for determining whether a corresponding position regarding a retention time is good or bad

A score for determining whether a corresponding position regarding a retention time is good or bad can be defined as follows, for example.

   i) The score at the upper leftmost point, that is, the score at the point where no corresponding relationships are determined, is defined as 0.

ii) When a corresponding relationship proceeds in one stage by selecting any one of the aforementioned cases (1), (2), and (3), the score that is determined for each of the cases (1), (2), and (3) is added to the immediately preceding score, so as to obtain a score indicating the new corresponding relationship. For example, such a score can be set for each of the cases (1), (2), and (3), as follows.

Case (1) (when the pathway proceeds in the diagonal direction in Figure 5):

In this case, $Z^{(1)}$ is allowed to correlate to $Z^{(2)}$ with regard to a certain retention time. Accordingly, in this case, as a score to be added, a value that reflects the degree of similarity or distance of the m/z parameter and the ionic intensity parameter between $Z^{(1)}$ and $Z^{(2)}$ can be set. A case where a score is defined as a degree of similarity will be described below. For example, in a case where although ionic intensity is detected on a certain m/z in $Z^{(1)}$, no ionic intensity is detected on the same m/z in $Z^{(2)}$, or in a case opposite thereto, a score can be set such that a certain value (penalty score) is subtracted. In addition, in a case where ionic intensity is found on a certain m/z in each of $Z^{(1)}$ and $Z^{(2)}$, a score can be set such that the absolute value of the difference between both ionic intensitys is multiplied by a certain coefficient and such that the thus calculated value (penalty score) is subtracted. Further, such a score may also be calculated using a function in which the greater the difference between both ionic intensitys, the lower the score that can be obtained.

Moreover, a deviation of retention time in $Z^{(1)}$ and $Z^{(2)}$ may also be reflected to such a score. For example, a score can be set such that a value (penalty score) calculated by multiplying the absolute value of the difference of retention time in $Z^{(1)}$ and $Z^{(2)}$ by a certain coefficient is subtracted. Such a score may also be calculated using a function in which the greater the difference in the retention times in $Z^{(1)}$ and $Z^{(2)}$, the lower the score that can be obtained.

When signals derived from a reference material correspond in $Z^{(1)}$ and $Z^{(2)}$, a special measure is preferably taken for the calculation of the score, as well as devising a calculation method, which will be described below. In particular, since it is strongly desired that these points correspond to each other between $Z^{(1)}$ and $Z^{(2)}$, when $Z^{(1)}$ is allowed to correlate to $Z^{(2)}$ as a result that signals derived from a reference material has been found both in $Z^{(1)}$ and $Z^{(2)}$, a large score is given. In contrast, when signals derived from a reference material are found only in either one, a large loss is given.

Cases (2) and (3) (when the pathway proceeds in the longitudinal or horizontal direction in Figure 5):

In this cases, no corresponding retention time is found in $Z^{(1)}$ and $Z^{(2)}$. Accordingly, in this case, a score is set such that a certain value (penalty score) is subtracted.

iii) Thus, scores are obtained stepwise from the upper leftmost angle to the lower rightmost angle of the lattices shown in Figure 5, and the score at the point of the lower rightmost angle finally becomes a score corresponding to the obtained corresponding position.

(d) Procedures for obtaining the optimum corresponding position regarding retention time

Basically, all possible corresponding positions may be listed up, and the score may be calculated for each of them, and the corresponding position with the greatest score may be selected from among the calculated scores. As stated above, since such a score is given by a recurrence formula, this is suitable for the "dynamic programming." That is, when the corresponding relationship of the $i^{th}$ retention time contained in the three-dimensional data $Z^{(1)}$ with the $j^{th}$ retention time contained in the three-dimensional data $Z^{(2)}$ is considered, the following 3 cases should be considered: (1) a case where the $i^{th}$ retention time of $Z^{(1)}$ is allowed to correlate to the $j^{th}$ retention time of $Z^{(2)}$, following the $i$-1st retention time contained in $Z^{(1)}$ and $j$-1st retention time contained in $Z^{(2)}$; (2) a case where there are no parameters of $Z^{(2)}$ corresponding to the retention time of $Z^{(1)}$, following the $i$-1st retention time contained in $Z^{(1)}$ and the $j^{th}$ retention time contained in $Z^{(2)}$; and (3) a case where there are no parameters of $Z^{(1)}$ corresponding to the retention time of $Z^{(2)}$, following the $i^{th}$ retention time contained in $Z^{(1)}$ and the $j$-1st retention time contained in $Z^{(2)}$. In all these cases, if the score at the immediately preceding stage is known, it becomes possible to calculate the $(i, j)^{th}$ score of $Z^{(1)}$ and $Z^{(2)}$.

Thus, among the three above cases (1), (2), and (3), only the best score and the pathway reaching it are recorded, and this step is continued from the starting point at the upper leftmost angle to the goal at the lower rightmost angle of the lattices shown in Figure 5. Thereafter, by reversely tracing the recorded pathway from the lower rightmost angle to the starting point, the optimum pathway, that is, the optimum corresponding position regarding the retention time in $Z^{(1)}$ and $Z^{(2)}$, can be obtained.

The method described above in procedures (a) to (d) can constitute a method for finding an optimum solution based on dynamic programming. The algorithm applicable in the present invention is not limited to such dynamic programming. That is to say, if the present algorithm is considered to be a more common searching method for optimizing an evaluation function of interest, it can also be implemented with other optimum searching algorithms. For example, the present algorithm can be implemented with other algorithms such as A* algorithm, genetic algorithm (GA), simulated annealing (SA), or nonlinear programming such as the steepest-descent method.

A method described in the procedures (a) to (d) above is a method based on what is called dynamic programming. From the viewpoint that this method is based on dynamic programming, this method is similar to the DTW method or the COW method. However, in such DTW or COW method, searching is performed under the same global constraint conditions in which the form of an evaluation function or a calculation method is based on an evaluation function involving Euclidean distance or correlation, in which comparison is made, directly on a column of time series data points, or on a time segments created by dividing the time at certain intervals, and in which the time 0 of two profiles is defined as a starting point and the stop time of each profile is defined as a goal. Moreover, such methods using DTW or COW comprise the alignment of a intensity profile to a time series profile basically shown as two-dimensional data, namely, a data set consisting of a time base and a signal intensity axis, by the nonlinear expansion of the time base.

Accordingly, in such methods using DTW or COW, such alignment operation is carried out, (1) using one or more sections having a specific value to a specific axis, or (2) by summarizing all the values along a specific axis. Such alignment operation could easily be conceived as a natural enhancement of these methods. For example, in such methods using DTW or COW, with regard to three-dimensional data consisting of retention time, m/z, and ionic intensity obtained as a result of the LC-MS analysis as well, the time base can be corrected by limiting m/z values to several specific m/z values, or summing up all the ionic intensitys along the retention time axis, as with the total ion chromatogram (TIC).

Nevertheless, the method described in the procedures (a) to (d) above differs from such enhanced DTW or COW method. The method described in the above procedures comprises directly subjecting multi-dimensional profiles, from which a dimension as a correction target (retention time axis) has been excluded, to a comparison, so as to expand the dimension as a correction target, thereby realizing the alignment of the multi-dimensional profiles. The method enhanced DTW or COW is problematic in that: (1) if a means for limiting to a specific segment is adopted, it is not guaranteed that the same results can be obtained as those in the case of aligning the entire profiles while keeping precision, there are no effective versatile means for selecting a specific segment, and there is a risk that the results are obtained in an arbitrary manner by performing alignment without such guarantee; and in that (2) a merit of using multiple dimensions to improve a resolution cannot be obtained by summarizing information, as with TIC. In contrast, the method described in the procedures (a) to (d) above has neither the aforementioned problem (1) nor (2), and enables the alignment of profiles at a high precision. In addition, this method also enables the alignment of profiles while maintaining high resolution regarding multi-dimensional data.

4. Role of reference material in data analysis

In the sample analyzing method of the present invention, high precision and high calculation efficiency can be achieved by incorporating information derived from a reference material into a calculation method using the present algorithm, as described below.

By adding a reference material before or at the midpoint of the process described in the above section "2. Sample analysis," a bias that is likely to be generated during the measurement and analysis processes can be corrected, and also, the aforementioned optimum corresponding position, that is, the alignment of profiles, can be carried out more precisely and more efficiently, using such information. That is to say, the following advantages can be obtained by using a reference material.

　　(1) The entire signal intensity is corrected by addition of a predetermined amount of reference material in advance, thereby enabling a quantitative comparison.
　　(2) Several reference materials can be used as landmarks, when parameters to be corrected (time base, etc.) are aligned.
　　(3) A certain extent of commonality in profile form facilitates the alignment of profiles.

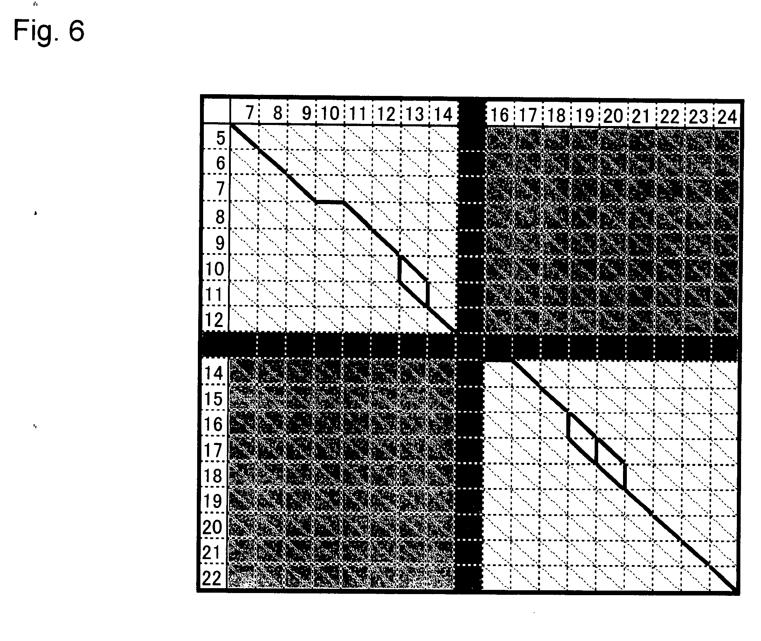

In order to achieve these advantages to the maximum extent in the process described in the above section "3. Data analysis," the calculation method can be modified as follows. That is, the calculation method can be modified, such that the pathway must pass through a peak portion of signals derived from a reference material in the aforementioned algorithm. More specifically, in the search for an optimum solution according to the aforementioned algorithm, the optimum pathway (bold line) from a starting point on the upper left-hand side to a goal on the lower right-hand side is searched in a lattice-form searching space shown in Figure 5. Herein, a constraint condition is established, in which if the point of retention time 15 on the longitudinal column and the point of retention time 13 on the horizontal row are both derived from a reference material, the pathway that can constitute a solution must pass through these points. By establishing such a constraint condition, pathways passing through a lower left portion and an upper right portion are excluded from a searching space that is partitioned with the lines representing column 15 and row 13. Thus, a space to be searched can be reduced (Figure 6).

Hence, by establishing a constraint condition regarding signals derived from a reference material thereby modifying the algorithm, the sample analyzing program of the present invention enables a more precise alignment of profiles, and also significantly improves processing efficiency.

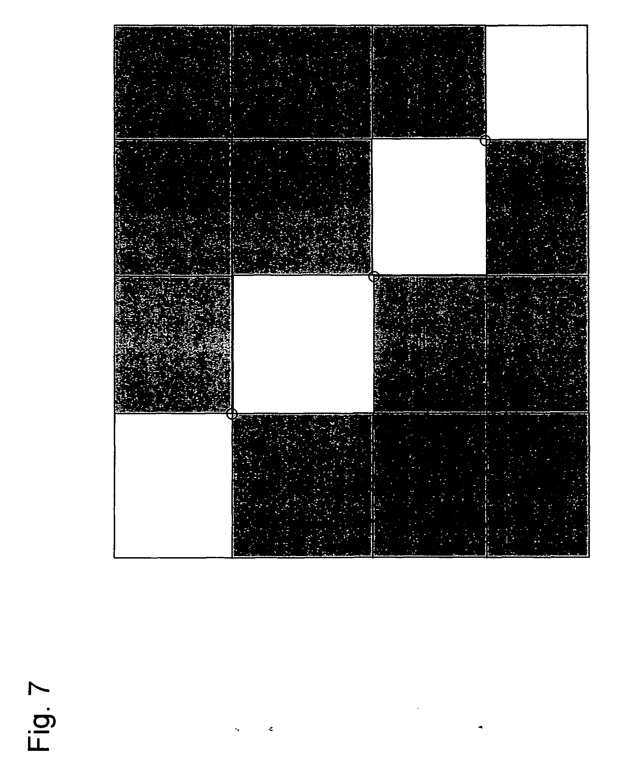

Moreover, since a searching space is further limited as the number of signals derived from a reference material increases, the alignment precision of profiles is further improved, and thus the improvement of efficiency can be anticipated. In reality, as shown in Figure 7, if a constraint condition is established in which the point indicated with a circle is defined as a point corresponding to a signal derived from a reference material, a region masked with a grey color is deleted from a searching space. When a time base is divided into the $n+1$ number of portions by the $n$ types of signals derived from a reference material, if such division is carried out at equal intervals as the best case, the searching space is reduced to at maximum $\dfrac{1}{n+1}$ .. When signals derived from a reference material are determined as constraint conditions, if reference materials are selected such that signals derived from the reference materials are equally broadly distributed, the effect of reducing a searching space can be obtained to the maximum extent.

Regarding a method of modifying the algorithm by limiting a searching space in order to enhance searching efficiency, another constraint condition, in which the searching space is limited to a space with a certain width before and after a diagonal from the starting point at the upper left corner to the goal at the lower right corner in the searching space shown in Figure 5, can also be conceived. However, in such a case, there is a risk that reliable prerequisite knowledge regarding the extent of limitation is not generally obtained. Moreover, in such a case, there is also a risk that when the starting point or the goal is largely deviated among multi-dimensional data to be compared, the optimum pathway to be obtained is run out of the limited space. For example, since the elution initiation time of chromatography may largely fluctuate, unless this time is reliably measured, the aforementioned method of limiting the searching space to a space with a certain width before and after a diagonal is not adequate.

In contrast, a method of modifying the algorithm such that a signal derived from a reference material is determined as a constraint condition has the point of time at which a reference material added into an analyte has appeared, and thus a signal derived from a reference material can constitute the most reliable reference point. Furthermore, since a searching space is reduced to, at maximum, approximately $\dfrac{1}{n+1}$ , it can be said that this method is excellent in terms of both reliability and efficiency.

Still further, by searching for such an optimum pathway on one or several partial spaces that are limited by signals derived from a reference material, a partial optimum alignment of profiles can be carried out. During this process, using the value of the aforementioned evaluation function as an index of the degree of profile alignment, the similarity (or distance) of profiles can be measured. In many cases, since major signals intensively appear in a limited time region, by limiting the searching space to a partial space and searching for the optimum pathway, so as to obtain the value of the evaluation function, the similarity (or distance) between profiles or samples generating such profiles can efficiently be obtained.

When a mean profile should be obtained by the alignment of profiles derived from a large number of samples, or when a sufficient amount of information regarding the attributes of samples has not been obtained in advance, first, the optimum pathway searching is conducted only in a partial space, so as to obtain the similarity (or distance) between samples, and then, the alignment of profiles is successively carried out in such an order, or grouping of samples can also be carried out. In particular, when profiles are aligned, the results may be changed depending on the order of being aligned. Thus, it is desired that profiles that exist closer to each other be aligned. This method is effective for conducing such processing.

5. Processing after data analysis

After obtaining the optimum alignment regarding two multi-dimensional data, a new value after correction is generated regarding the corrected parameter. In particular, when the retention time of chromatogram has been corrected, the retention time after correction is obtained. Examples of a method of obtaining the retention time after correction may include: a method of defining one of the two multi-dimensional data to be aligned as a reference data, and allowing the retention time in the other multi-dimensional data to correspond to the retention time of the above-described reference data (asymmetrical type); and a method of correcting both of the two multi-dimensional data to be aligned (symmetrical type). It is particularly preferable that the retention time after correction be obtained by the symmetrical-type method.

When two multi-dimensional data are aligned by the asymmetrical-type method to obtain the retention time after correction, in order to align to the retention time axis in the reference data, the retention time in the reference data is directly used for corresponding points. When there are obtained no points to which the reference data correspond, the retention time after correction can be determined by interpolation using corresponding points before and after the point.

However, in order to align two multi-dimensional data by the asymmetrical-type method, it is necessary to determine in advance, which one of multi-dimensional data is used as reference data. For example, it is also possible to use a blank containing only a reference material as reference data. In this case, however, there is a high possibility that the profile of the multi-dimensional data that has initially been used for alignment significantly affects on the results.

In contrast, when two multi-dimensional data are aligned by the symmetrical-type method to obtain the retention time after correction, regarding portions in which corresponding points are obtained between the two multi-dimensional data, the arithmetic mean of each retention time is adopted. When only the data point of either one of the two multi-dimensional data is obtained, the retention time after correction is obtained by interpolation from a group of retention times after correction of the closest corresponding points before and after the point. When it is impossible to make such correction by interpolation, based on the retention time after correction of the closest corresponding point, the retention time after correction can be obtained by extrapolation using, as a coefficient, a mean time scale on the data set as a whole.

In this case, it may also be possible that the similarity (distance) among all multi-dimensional data has previously been calculated, and that profiles that exist close to each other be successively aligned, as stated above.


6. Output processing

A method of outputting profiles obtained in the above section "5. Processing after data analysis" may include the following (1) and (2).

    (1) All profiles, including portions in which no corresponding points are obtained, are outputted.
    (2) Only the corresponding points are outputted.

An output method may appropriately be selected from the two above method, depending on the purpose of using the analyzing method of the present invention. For example, when the purpose of use is to obtain a mean value from the results obtained by measuring a single sample several times to cancel measurement error, or when a representative profile is obtained from several samples measured under extremely similar conditions, the output method described in (2) above is effective. Since the outputted profile is limited to a common portion according to the output method described in (2) above, the size of data can be reduced, so as to enhance processing efficiency.

In contrast, when a difference between groups of different samples is detected for example, the output method described in (1) above must be applied. When the output method described in (1) above is applied, the size of data generally increases, but the loss of information does not occur.

Moreover, when the output method described in (1) above is selected, alignment can also be carried out, while common profiles are emphasized. In this case, when a new point is allowed to correspond to the point in the previous alignment process, a new term for meliorating the score of an evaluation function may be established in the evaluation function, so as to adjust such that points can be aligned at the same point as much as possible. That is to say, for example, such a new term as $-\mu \cdot \delta_m(i,j)$ is added to the end of the evaluation function represented by the aforementioned formula (I), and the thus obtained new evaluation function is used to calculate the evaluation score. When such an evaluation function is used, if a new point can correspond to the previously aligned point, $\delta_m(i, j)$ is defined as 1. In other cases, $\delta_m(i, j)$ is defined as 0.

In the sample analyzing program of the present invention, information is outputted in the following forms:

- Information regarding new points obtained by alignment processing;
- Information regarding points of the corresponding input data set 1 (one type of multi-dimensional data); and
- Information regarding points of the corresponding input data set 2 (the other type of multi-dimensional data).

Information is outputted in the form that these types of information are repeated for the number of data points obtained as a result of the alignment processing. However, when there are no corresponding points, information regarding input data set 1 or 2 does not exist. Thus, the outputted information also includes the points of the corresponding input data set. As described later in examples, it becomes possible to determine the type of the original multi-dimensional data from which each point of the finally obtained aligned profile is derived. Further, it is also possible to output additional information other than the aforementioned information, as necessary.

The thus obtained profile after alignment may further be subjected to a process of summarizing or quantizing several parameters, as necessary. For example, in particular when all points are outputted as described in (1) above, the resolution of the time base may become unnecessarily higher than the desired level. In such a case, it is convenient for the subsequent processes to combine points extremely adjacent to each other on the time base into a single point.

The intensity of the thus combined point can be substituted by addition of individual points regarding intensity before such combination. Likewise, also on the m/z axis, points that are adjacent to each other to the extent that it exceeds necessary resolution can be combined. This operation may be carried out for every alignment processing. Otherwise, it may also be carried out only once at the last stage, after necessary alignment has been first conducted.

7. Normalization of ionic intensity and reference material

In the sample analyzing method of the present invention, it is preferable to normalize the measured ionic intensity prior to the process described in the above section "3. Data analysis." Normalization of ionic intensity will be described below. However, a method for normalizing ionic intensity is not limited thereto.

Specifically, first, the RAW file obtained as a result of the LC-MS analysis is converted into a text file, using utility software of Xcalibur™, for example. Subsequently, the following series of data processing steps are carried out by the program written in the C language and the Perl language.

(1) Signals, the ionic intensity of which is a certain value or less (for example, $10^2$ or less), are eliminated, so as to eliminate data with noise level.

(2) If necessary, data points are summarized in order to reduce of the processing time. Specifically, for example, the m/z value and retention time in the original data are rounded off, such that the m/z is set in increments of 1 and such that the retention time is set in increments of 0.2. Data points with the same values (m/z, retention time) are added together and accumulated.

(3) Signals derived from a reference material are identified based on the previously measured m/z value and retention time, and the total measurement values are divided by the ionic intensity value thereof for normalization. During this process, a method using, as the ionic intensity value of a reference material, a representative value such as a mean value of multiple signals derived from one or more reference materials, or a method using the most stable signal value after the stability of signals has previously been examined by a preliminary experiment or the like, may be applied.

More specifically, when chicken egg-white lysozyme is used as a reference material for example, a signal with an m/z value of approximately 715 and a signal with an m/z value of approximately 877 can be used as reference signals. When searching through the measurement data of samples, m/z is set at the actual value $\pm 1$, and regarding the retention time, the signal with m/z of 715 (715 $\pm 1$) is searched through an early elution signal group, and the signal with m/z of 877 (877 $\pm 1$) is searched through a late elution signal group, thereby searching for a signal derived from the reference material. Thereafter, correction may further be carried out, such that the signal intensity derived from the reference material is corrected to be $10^7$ by multiplying the obtained value by $10^7$.

Moreover, when a peptide itself is used as a reference material, for example, when peptide T (Ala-Ser-Thr-Thr-Asn-Tyr-Thr) and β-casomorphin-7 (Tyr-Pro-Phe-Pro-Gly-Pro-Ile) are used as reference materials, a signal with m/z of approximately 859 and a signal with m/z of approximately 791 can be used as reference signals, respectively. The former peptide is relatively hydrophilic, and the latter is hydrophobic. In reverse phase chromatography used for separation due to retention time in the present analysis method, the value of the former retention time is low, and that of the latter retention time is higher than it. The retention times of peptides derived from the majority of samples exist between the retention times of these two types of peptides. With regard to the measurement data of samples, m/z is set at the actual value $\pm 1$. Regarding the retention time, an approximate value has previously been estimated from the chromatogram obtained by the measurement of only a reference material, and the actual value is searched within a certain range around the estimated value, thereby finding out a signal derived from the reference material.

When a peptide is used as a reference material in the present analysis method, in order to suppress detection of signal noise to the minimum extent, it is important not to contain substances other than the above peptide (for example, contaminants). Accordingly, it is preferable to use chemically synthesized peptide molecules, rather than products obtained by extraction and/or purification from natural products. In addition, as properties of such a peptide molecule, it is important that the structure thereof be stable or that such a peptide molecule do not become insoluble under predetermined measurement conditions. It is preferable that amino acid residues constituting a peptide molecule do not contain amino acid residues that are easily oxidized, such as methionine, tryptophan, or histidine, and that they do not contain two or more basic functional groups. In particular, the latter characteristic is important to prevent detection of multiple ion signals with different valences in a single reference material, when the electrospray ionization method that generates multivalent ions in its principle is used in MS as a measurement means.

When a peptide fragment produced from a protein by hydrolysis or chemical cleavage is used as a reference material, it is preferable that the intensity of peptide ion signals derived from the above protein, other than the signal adopted as a reference material, be as low as possible.

The measurement ionic intensity value can be normalized by the aforementioned steps (1) to (3), and thus, ionic intensity can quantitatively be compared for multiple samples. It is to be noted that the measured ionic intensity value should be normalized prior to the aforementioned correction of the retention time.

8. Comparative analysis among samples

According to the sample analyzing method of the present invention, using three-dimensional data consisting of m/z, the normalized ionic intensity, and the corrected retention time, various types of components, such as a group of proteins contained in a sample, can be analyzed on a computer. Specific examples of such component analysis may include: (a) an addition method; and (b) a subtraction method.

(a) Addition method

As stated above, since parameters of retention times are appropriately corrected in a plurality of three-dimensional data obtained by the sample analyzing method of the present invention, the corresponding relationship between data points can exactly be determined. Thus, in the plurality of three-dimensional data, the normalized ionic intensity values of data points can be added together.

(b) Subtraction method

As with the above "(a) Addition method," since the corresponding relationship between data points can exactly be determined in a plurality of three-dimensional data obtained by the sample analyzing method of the present invention, a difference between the normalized ionic intensity values of data points can be obtained by subtraction.
Thus, since the plurality of three-dimensional data obtained by the sample analyzing method of the present invention can be subjected to addition or subtraction, the following approach for component analysis can be realized on a computer.

(1) Application for accumulation of experimental data:

Even in a case where a sample derived from a single sample is divided into multiple fractions and the fractions are then measured for measurement convenience, the corresponding relationships between data points can exactly be obtained in the three-dimensional data obtained from each of the multiple fractions. Accordingly, according to the aforementioned addition method, all the three-dimensional data can be added together. By such addition, the analysis of components contained in the original sample as a whole or the like can be carried out.
When such accumulation is carried out, other than the method of adding together all the multiple fractions and combining them into a single profile, a method of summarizing a predetermined number of several profiles that are adjacent to one another may also be applied. In this case, the entire sample is divided into an $n$ number of fractions. Among such fractions, when an m number of fractions that are adjacent to one another are combined, an $n-m+1$ number of profiles are obtained. In such a case, the obtained profiles other than the fractions corresponding to each other are treated separately, and the operation described below is performed thereon. In any case, even in a case where multiple fractions are obtained by the multi-dimensional fraction method, it is very rare that they are completely separated. In many cases, there may be carry-over in multiple fractions. Thus, such an accumulation operation is required.

(2) Application for obtainment of a representative value from the measurement results of multiple samples:

Even in a case where multiple samples derived from different samples are measured, the corresponding relationships between data points can exactly be obtained in a plurality of the obtained three-dimensional data according to the analyzing method of the present invention. Accordingly, all the three-dimensional data can be added together according to the aforementioned addition method. Thereafter, an arithmetic mean can be obtained by dividing the total sum of the obtained three-dimensional data by the number of samples. As necessary, a weight is established for each sample, and a weighted mean that reflects the above weight can also be calculated.
According to this method, a representative value of multiple samples belonging to the same range can also be calculated.

(3) Application for obtainment of a difference between the measurement results of two samples

For example, even in a case where samples collected from a single sample, which is however in different states, are measured, the corresponding relationships between data points can exactly be obtained in the two obtained three-dimensional data. Accordingly, a difference between the two three-dimensional data can be obtained according to the aforementioned subtraction method. By this method, the change in components contained in a sample caused by the change in the state can be analyzed.

Moreover, for example, a representative value such as an arithmetic mean is obtained from each of two groups containing multiple samples in accordance with the method described in (1) above, and thereafter, a difference between these representative values of the two groups can be obtained. The significance of the obtained difference is tested by a statistical test or the like, so as to identify a component specific to each group.

The component analysis approaches described in (1) to (3) above may be applied, either using either database for storing multiple three-dimensional spectrum data obtained by the sample analyzing method of the present invention, or using both data stored in the above database and the actually obtained data. In both cases, the component analysis approaches described in (1) to (3) above can easily be realized using a computer.

Thus, with regard to group-specific signal components obtained by the sample analyzing method of the present invention, a group of proteins from which the above signal is derived can be identified by the tandem MS analysis or the like, in which the range is limited to the obtained signal region. That is to say, in the sample analyzing method of the present invention, when peptide molecular ions with a specific m/z value are detected during the analysis of a sample by LC-MS, the CID spectrum of the above ions can be measured.

Thereafter, the obtained CID spectrum is inputted in a computer, and searching is carried out against protein sequences obtained from the protein primary structure database, the genomic sequence database, or the cDNA sequence database, using database searching software. When a significant hit score is obtained as a result of such database searching, information regarding proteins, amino acid sequences, or the like registered in the database can be obtained, and the obtained information is allowed to correlate to the obtained CID spectrum.

For example, by measuring a CIP spectrum regarding a signal identified as a component specific to each group in the component analysis approach described in (3) above, a protein group indicated by the above signal can be identified.

Examples

The present invention will be described more in detail below in the following examples. However, the examples are not intended to limit the technical scope of the present invention.

Example 1

In Example 1, a peptide sample obtained by mixing protease digests of proteins having amino acid sequences that have already been known was measured by LC-MS. Thereafter, the algorithm of the present invention was applied to a three-dimensional profile consisting of retention time, m/z value, and ionic intensity, which was obtained by the above measurement, thereby obtaining the characteristics of the measured peptide sample in a quantitative manner. In addition, in Example 1, as a model experiment for comparative quantification, each of several types of peptide samples obtained by mixing protease digests of proteins having amino acid sequences that have already been known was measured by LC-MS, and the sample analyzing method of the present invention was then applied to the obtained data, so as to compare three-dimensional profiles. As a result, it was found that the difference in the type of proteins contained in each peptide sample is detected.

Preparation of peptide sample

24 types of trypsin-digested products of proteins as listed below were prepared as peptide samples used in the present example: (1) bovine chymotrypsinogen; (2) bovine catalase; (3) bovine carbonic anhydrase; (4) bovine apotransferrin; (5) bovine carboxypeptidase A; (6) bovine serum albumin; (7) horse cytochrome c; (8) porcine γ-immunoglobulin; (9) bovine hemoglobin; (10) horse myoglobin; (11) bovine β-lactoglobulin; (12) bovine deoxyribonuclease; (13) rabbit glyceraldehyde-3-phosphate dehydrogenase; (14) chicken conalbumin; (15) horseradish peroxidase; (16) *Bacillus subtilis* α-amilase; (17) horse glutathione S-transferase; (18) bovine glutamate dehydrogenase; (19) bovine lactoperoxidase; (20) Aspergillus amyloglucosidase; (21) rabbit phosphorylase B; (22) bovine β-galactosidase; (23) rabbit lactate dehydrogenase; and (24) chicken egg-white lysozyme. These digested products were purchased from Michrom BioResources.

These 24 types of trypsin-digested products of proteins were mixed as follows, so that the total 3 types (group A to group C) of peptide samples were prepared.

Group A: 20 types of trypsin-digested products of proteins described in (1), (2), and (7) to (24) above. Proteins characteristic of group A are (1) and (2). Group B: 20 types of trypsin-digested products of proteins described in (3), (4), and (7) to (24) above. Proteins characteristic of group B are (3) and (4). Group C: 20 types of trypsin-digested products of proteins described in (5) to (24) above. Proteins characteristic of group C are (5) and (6). Three samples were prepared for each of the above groups.

LC-MS analysis

In order to obtain the three-dimensional profile of each peptide sample, the peptide sample was analyzed by the following operation using the following device (Kawakami T. et al., Jpn. J. Electrophoresis 44: 185-190 (2000)). First, the peptide sample that had been concentrated under a reduced pressure was dissolved in 45 µl of a solvent consisting of trifluoroacetic acid, acetonitrile, and water at a mixing ratio of 0.1 : 2 : 98. The obtained solution was defined as a dissolved solution.

Subsequently, using the autosampler PAL LC-1™ manufactured by CTC Analytics, 20 µl of the dissolved solution was introduced into the MAGIC MS™ C18 capillary column (inside diameter: 0.2 mm; length: 50 mm; grain diameter: 5 µm; pore size: 200 angstroms) manufactured by Michrom BioResources. The peptide was eluted using the MAGIC 2002™ HPLC system (Michrom BioResources). During this process, HPLC mobile phase A was a solvent produced by mixing formic acid, acetonitrile, and water at a volume ratio of 0.1 : 2 : 98. In contrast, the mixing ratio of components in mobile phase B was 0.1 : 90 : 10. The concentration of mobile phase B was increased from 5% to 85% in a linear gradient manner, so as to continuously elute peptide fragments. The flow rate was set at approximately 1 µl/min. The LC eluate was directly introduced into an ion source in the LCQ™ ion trap mass spectrometer (ThermoQuest) via the PicoChip™ needle (inside diameter: 20 µm) manufactured by New Objective. Fine adjustments were made for the distance of a NanoESI needle from a heating capillary. The spray voltage was not applied to the needle, but it was directly applied to the eluate. Gas was not used for spaying. The spray current was set at 3.0 mA. Such spraying was conducted on each group 3 times, so as to obtain aggregates of three-dimensional parameters corresponding to the samples, namely, 9 data sets in the total 3 groups. These data sets were defined as A1, A2, A3 (group A), B1, B2, B3 (group B), and C1, C2, C3 (group C).

A file containing a three-dimensional parameter aggregate was converted into a text file using Xcalibur™ utility software. The following data processing steps (1) to (5) were carried out by a program written in the C language and the Perl language.

(1) Signals with ionic intensity of $10^2$ or less were eliminated, so as to eliminate data with noise level.

(2) Data points were combined for reduction of the processing time. Specifically, the m/z value and retention time in the original data were rounded off, such that the m/z was set in increments of 1 and such that the retention time was set in increments of 0.2. Data points, which were designated by m/z and retention time with the same values, were added together and accumulated.

(3) Signals derived from chicken egg-white lysozyme as a reference material were identified. That is to say, a data point with the highest ionic intensity was searched in a range before and after the m/z value and retention time value of a reference material that had been actually measured in a preliminary experiment. Thereafter, data points, the ionic intensity of which was monotonously decreased and was 0 or greater, were picked up around the above data point. These data points were considered to be data points due to signals derived from the reference material. The total ionic intensity value of the signals derived from the reference material was considered to be the total sum of ionic intensitys of data points that were considered to be the signals derived from the reference material. Specifically, a signal with an m/z value derived from chicken egg-white lysozyme that was approximately 715, and a signal with an m/z value therefrom that was approximately 877, were used as reference signals. When these signals derived from the reference material were searched through the measurement data of samples, regarding m/z, searching was carried out within the range of the actual value ±1. Regarding the retention time, the signal with m/z of 715 was searched within the range between 6 and 16 minutes, and the signal with m/z of 877 was searched within the range between 13 and 23 minutes.

(4) The ionic intensity of each signal was divided by the total ionic intensity value of the obtained signals derived from the reference material, and the obtained value was then multiplied by $10^7$, so as to correct the signal intensity derived from the reference material to $10^7$.

(5) For the sake of convenience, the retention time axis was subjected to linear transformation, so that the peak position of the signal with m/z of 715 and that of the signal with m/z of 877 became 10 minutes and 20 minutes, respectively, with regard to the retention time.

Subsequently, a representative point of the three-dimensional profiles obtained from 3 samples in each of groups A, B, and C was obtained. That is, as stated above, samples belonging to the same group were summarized. The ionic intensitys at the points where m/z and retention time overlapped were added together and accumulated.

In addition, the score used in the present example was calculated in such a manner that the higher the score, the better the result that can be obtained. Coefficients used in calculation formulas are as follows. As a difference in the ionic intensity, the absolute value of the difference in the common logarithm that was multiplied by -1 was used. As a difference in the retention time, the absolute value of the difference that was multiplied by -1,000 was used. In addition, when signals of data points corresponding between groups were both derived from the reference material, 50,000 points were added. When there were no corresponding retention time points in either one group, 5,000 demerit points were received. In the present example, these points were simply added to obtain a score.

Subsequently, as stated above, differences were obtained between groups A and B, groups B and C, and groups C and A. The significance of the obtained difference was examined by the t-test using a two-sided test with a significance level of 0.1%.

Three-dimensional data with the corrected retention time were compared. As a result, peptide molecular ions with the m/z values described below were detected in each of groups A, B, and C, as signals specific to each group.
Group A: 495, 524, 546, 560, 671, 696, 779, 845, 871, 908, 962, etc.
Group B: 451, 464, 509, 513, 546, 555, 583, 585, 626, 635, 649, 653, 701, 720, 723, 740, 741, 753, 768, 789, 819, 821, 847, 873, 886, 922, 928, 952, 966, 973, 978, 1057, 1230, etc.
Group C: 636, 670, 674, 679, 683, 718, 734, 735, 770, 824, 870, 918, etc.

Moreover, in the present example, in order to obtain the CID spectrum of the peptide molecular ions detected as specific signals, each sample was subjected to the LC-MS/MS analysis. Analytical conditions were the same as those described above, with the exception that the following operation was carried out. That is to say, for the LC-MS/MS analysis, the measurement conditions for the ion-trap mass spectrometer were changed. That is, the measurement conditions regarding an ion-trap mass spectrometer were changed, such that when peptide molecular ions with the m/z values as listed above were detected, the CID of the ions were definitely carried out, and the measurement of samples was then carried out.

The CID spectrum obtained from each peptide molecular ion was searched against the SWISS-PROT protein sequence database, using MASCOT™, database searching software from Matrix Science. As a result, 2 types of proteins in each group that had been added as peptides derived from proteins specific to each group (namely, the proteins described in (1) and (2) above in group A; the proteins described in (3) and (4) above in group B; and the proteins described in (5) and (6) above in group C), that are, the total 6 types of proteins, were identified with significant hit scores. These results show the validity of the sample analyzing method applied in the present example.

Example 2

In Example 2, a sample was produced by mixing a protein mixture with a certain concentration into another protein sample with a different concentration. The thus obtained sample was then digested with protease, and the obtained digested product was measured by LC-MS. Thereafter, the method of the present invention was applied to the obtained three-dimensional data consisting of retention time, m/z value, and ionic intensity. Two three-dimensional data obtained by measurement of samples with different concentrations were compared, so as to detect signals that fluctuated in a quantitative manner. This shows that a substance that fluctuates in a quantitative manner can be detected by the method of the present invention.

Sample and preparation thereof

The following 6 types of trypsin-digested products of proteins were prepared as peptide samples in the present example: (1) bovine catalase; (2) bovine β-lactoglobulin; (3) bovine lactoperoxidase; (4) horse glutathione S-transferase; (5) horseradish peroxidase; and (6) bovine serum albumin. These proteins were purchased from Sigma.

These proteins were allowed to react with porcine trypsin (purchased from Promega) in an aqueous solution, so as to obtain trypsin-digested products.

The trypsin-digested products of the 6 types of proteins were mixed as follows, so as to prepare the total 7 types of peptide samples.

[1] (1) to (5) 500 femtomoles per measurement, and (6) 0 femtomole;
[2] (1) to (5) 500 femtomoles per measurement, and (6) 10 femtomoles;
[3] (1) to (5) 500 femtomoles per measurement, and (6) 50 femtomoles;
[4] (1) to (5) 500 femtomoles per measurement, and (6) 100 femtomoles;
[5] (1) to (5) 500 femtomoles per measurement, and (6) 500 femtomoles;
[6] (1) to (5) 500 femtomoles per measurement, and (6) 1 picomole;
[7] (1) to (5) 500 femtomoles per measurement, and (6) 5 picomoles.

The samples in each group were prepared for 5 measurements. Thereafter, as reference materials, peptide T and β-casomorphin-7 were added to the aforementioned each sample at amounts of 10 picomoles and 1 picomole, respectively.

LC-MS analysis

In order to obtain the three-dimensional data of each peptide sample, the peptide sample was analyzed by the following operation using the following device (Kawakami T. et al., Jpn. J. Electrophoresis 44: 185-190 (2000)). First, the peptide sample that had been concentrated under a reduced pressure was dissolved in 45 μl of a solvent consisting of trifluoroacetic acid, acetonitrile, and water at a mixing ratio of 0.1 : 2 : 98. The obtained solution was defined as a dissolved solution.

Subsequently, using the autosampler PAL LC-1™ manufactured by CTC Analytics, 20 μl of the dissolved solution was introduced into the MAGIC MS™ C18 capillary column (inside diameter: 0.2 mm; length: 50 mm; grain diameter: 5 μm; pore size: 200 angstroms) manufactured by Michrom BioResources. The peptide was eluted using the MAGIC 2002™ HPLC system (Michrom BioResources). During this process, HPLC mobile phase A was a solvent produced by mixing formic acid, acetonitrile, and water at a volume ratio of 0.1 : 2 : 98. In contrast, the mixing ratio of components in mobile phase B was 0.1 : 90 : 10. The concentration of mobile phase B was increased from 5% to 85% in a linear gradient manner, so as to continuously elute peptide fragments. The flow rate was set at approximately 1 μl/min. The LC eluate was directly introduced into an ion source in the LCQ™ ion trap mass spectrometer (ThermoQuest) via the PicoChip™ needle (inside diameter: 20 μm) manufactured by New Objective. Fine adjustments were made for the distance of a NanoESI needle from a heating capillary. The spray voltage was not applied to the needle, but it was directly applied to the eluate. Gas was not used for spaying. The spray current was set at 3.0 mA. Moreover, in order to increase the number of scanning with a mass spectrometer, the Turbo Scan method was applied. This measurement was carried out 5 times for each group, so as to obtain aggregates of three-dimensional parameters corresponding to the samples, namely, 35 data sets in the total 7 groups. Figure 1 shows examples of the obtained profiles.

Data processing

A file containing the three-dimensional data was converted into a text file using Xcalibur™ utility software. The following data processing steps (1) to (7) were carried out by programs written in the C language, the C++ language, and the Perl language.

(1) Signals with ionic intensity of $10^2$ or less were eliminated, so as to eliminate data with noise level.

(2) Signals derived from peptide T and β-casomorphin-7 used as reference materials were identified. That is to say, a data point with the highest ionic intensity was searched in a range before and after the m/z value and retention time value of the reference materials that had been actually measured in a preliminary experiment. From among signals within a certain range around the data point, signals within a intensity range in the Gaussian distribution having the above data point as a vertex were picked up. All these selected signals were considered to be signals derived from the reference materials. More specifically, the limit of m/z was set at the actual value 858.9 or 791.0 ±2, and the limit of retention time was set at 9 minutes or 25 minutes ±6. Within these limits, searching was carried out by the aforementioned procedures. Accordingly, the used signals derived from the reference materials were two signals, one of which exists close to m/z of 858.9 and retention time of 9 minutes, and the other of which exists close to m/z of 791.0 and retention time of 25 minutes. When ionic intensity is corrected, all the signal intensities derived from the reference materials were added together, and the obtained value was normalized to be $10^9$. In addition, as a constraint point on the dynamic programming searching space during the time base correction, each one point for giving a intensity peak was selected from the aforementioned two reference material signals, and thus the total two points were selected.

(3) Each of the 7 types of samples with different BSA concentrations was measured 5 times. In order to average profiles obtained as a result of such measurement, the profile alignment program of the present invention was used to obtain a representative profile of each of the 7 groups of samples. The following evaluation function parameters were used for alignment.

In the aforementioned formula (I), penalty α that depends on the difference (absolute value) on the time base was set at 1.0, penalty β that depends on the difference on the signal intensity was set at 0.1 (wherein signal intensity was defined as an absolute value of the difference after conversion into a common logarithm), bonus point σ for the correspondence of the points was set at 0, penalty π for the non-correspondence of the points was set at 100, and bonus point $\theta(i,j) = S_m$ for the correspondence of the reference material-derived signals was set at 1,000. In addition, an output option regarding aligned profiles was limited to only corresponding points.

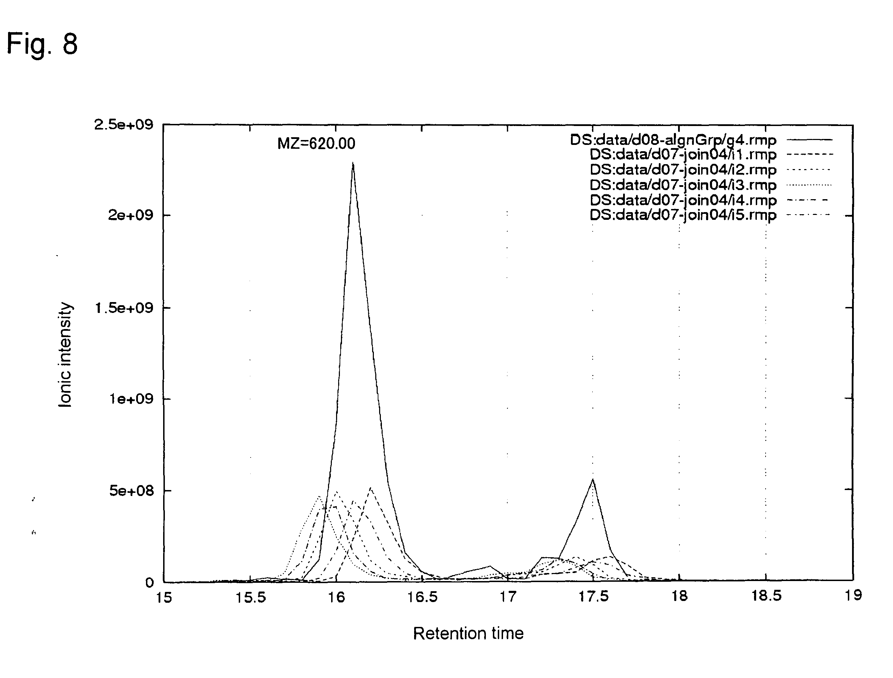

Figure 8 shows a chromatogram indicating m/z of approximately 620.0 and a residence time between 15 and 19 minutes from among the profiles obtained from the results of measurement performed 5 times on the sample [5] wherein BSA concentration was 500 femtomoles. Five grey waveforms that were slightly deviated one another on the time base show the results of measurement performed 5 times before alignment processing. The solid line shows the results obtained by subjecting these 5 waveforms to alignment processing and adding together all the signals. As shown in the figure, the fluctuation on the time base was corrected, so that the measurement results could be treated as a single large peak.

(4) Subsequently, in order to pick up signals that significantly fluctuate among groups, the representative profiles of the 7 groups obtained in the aforementioned process were further subjected to alignment processing, so as to finally obtain a single summarized profile. The following evaluation function parameters were used for alignment. In the aforementioned formula (I), penalty $\alpha$ that depends on the difference (absolute value) on the time base was set at 1.0, penalty $\beta$ that depends on the difference on the signal intensity was set at 0.1 (wherein signal intensity was defined as an absolute value of the difference after conversion into a common logarithm), bonus point $\sigma$ for the correspondence of the points was set at 0, penalty $\pi$ for the non-correspondence of the points was set at 100, and bonus point $\theta(i,j) = S_m$ for the correspondence of the reference material-derived signals was set at 1,000. In addition, an output option regarding aligned profiles involves all the points including non-correspondence.

(5) In order to summarize data points while maintaining resolution necessary and sufficient for the comparison between samples, among points within the range consisting of a m/z range $\pm 0.75$ (absolute range $\pm 2$) and a retention time range $\pm 1.25$ (absolute range $\pm 4$), points satisfying the following conditions were summarized. That is to say, all the data points within the aforementioned range were checked in decreasing order of signal intensity, and when it was determined that several points were included in the range in the Gaussian distribution having a peak signal as a vertex, they were summarized.

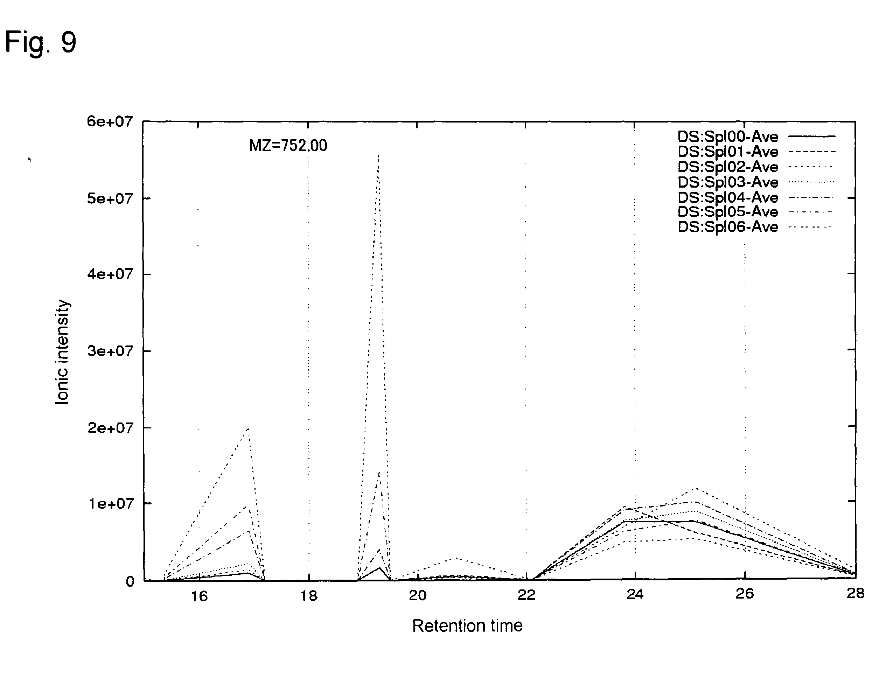

Figure 9 shows an example of the profile obtained after time base correction and summarization, of each of 7 types of samples with different BSA concentrations. In the figure, the section cut by a specific m/z value (which was 752 in this example) was expressed as a chromatogram, in which the intensity was plotted along the time base. With regard to summarized signals around 17 minutes and 19 minutes, sample 06 with the highest BSA concentration (which is represented by "DS: Spl 06-Ave" in the figure; BSA: 5 picomoles) exhibited the highest peak, and the level of the peak was then continued in the order of Spl 05, Spl 04, .... Thus, it can be determined that these are signals derived from BSA. On the other hand, moderate peaks around 25 minutes were observed in all the samples. Thus, it can be determined that these are derived from common substances other than BSA or background.

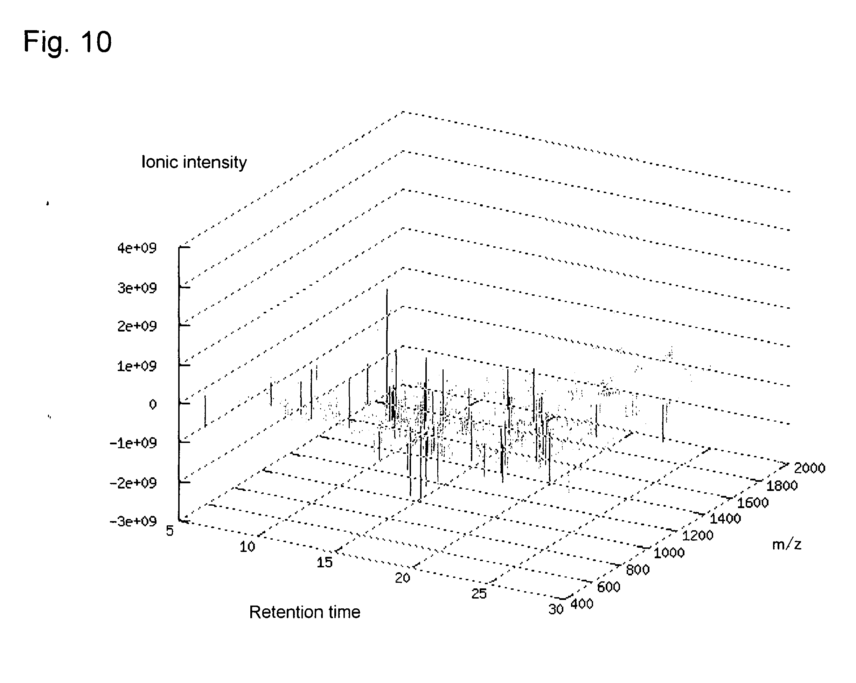

(6) By successively performing the above-described processes in the order of (5), (4), and (3), using the aforementioned summarized profile as a starting point, it becomes possible to trace, from which of the measurement results (7 groups x 5 measurements) the point on the finally obtained summarized profile was derived. By returning to the measurement results data for each point on the summarized profile, the profile of each group or a difference profile between groups can be obtained. Figure 10 shows the difference profile between sample [5] with BSA concentration of 500 femtomoles and sample [1] with BSA concentration of 0 femtomole. The line that is extended upward from the m/z - retention time plane indicates a signal that was significantly observed in sample [5]. In contrast, the line that is extended downward indicates a signal that was significantly observed in sample [1].

(7) The summarized profile obtained from sample [1] containing no BSA was used as a reference (reference profile). From each of the summarized profiles obtained from the remaining 6 types of samples [2] to [7] with different BSA concentrations (target profiles), data points satisfying the following conditions were obtained. (1) With regard to points on the target profiles, data are obtained from all the 5 measurements. (2) The signal intensity of points on the summarized target profiles is $10^6$ or greater. (3) The difference obtained by subtracting the intensity of the corresponding point on the reference profile from each point on the target profiles is 0 or greater. (4) The null hypothesis that the difference in signal intensity between each point on the target profiles and the corresponding point on the reference profile is less than 10,000 is rejected by the results of the one-sided t-test with a significance level of 0.5%.

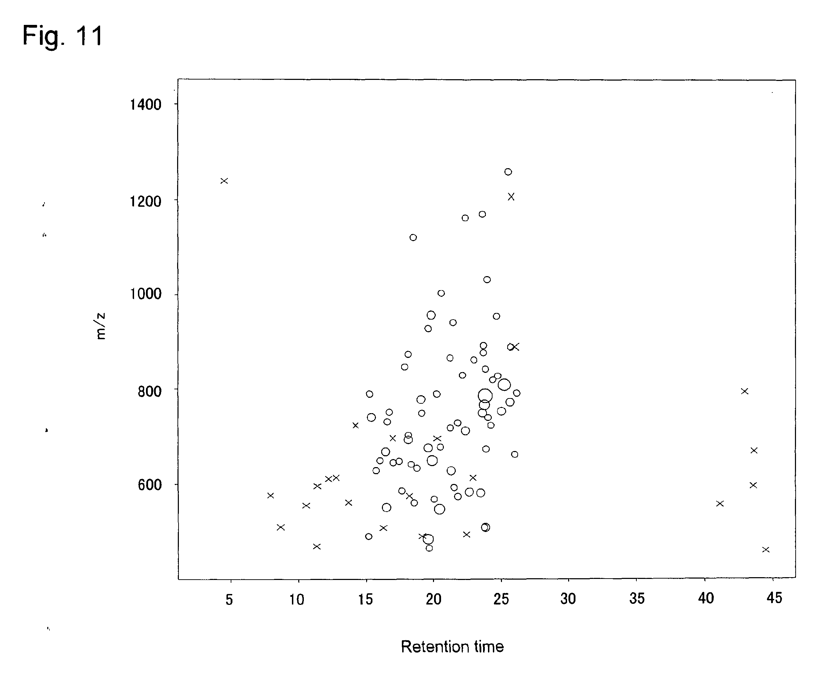

Figure 11 shows signals selected under the aforementioned conditions in the case of sample [5]. In the figure, 127 signals remained as those satisfying the aforementioned conditions. The size of a plot mark indicates the signal intensity of the profile of sample [5]. Plot mark ○ indicates the signal that was allowed to correlate to the BSA signal in the process described later. Plot mark x indicates the signal that was not allowed to correlate to the BSA signal.

These 127 signals were checked against the results of an MS/MS experiment with BSA digested products, which was performed separately. As a result, 103 signals thereof were matched with the signals derived from BSA. That is to say, it can be said that at least 81% of the detected 127 signals were those that had really been required.

Likewise, signals selected under the same above conditions from the signals detected in other samples with different BSA concentrations were checked against the signals derived from BSA. As a result, it was determined that 65%, 64%, 75%, 81% (as described above), 76%, 48% of signals from target profiles [2] to [7], respectively, were signals derived from BSA. The correct answer rate of the final sample [7] (BSA concentration: 5 picomoles) was low. This is because the threshold value of the profile was changed due to the presence of a large number of signals with high intensity derived from BSA with high concentration, and because false positive signals thereby increased. In reality, when selection condition (2) was adjusted to $3 \times 10^6$ such that almost the same number of signals as those of other samples with different concentrations were selected, the correct answer rate became 75%.

Reduction in the searching space in dynamic programming is one of measures taken in the present invention. The effect thereof was evaluated by the actual measurement of the CPU time. Taking the case of aligning the results of measurement performed 5 times on sample [5] as an example, the reduction effect of 43% to 45% was obtained with respect to the CPU time.

Herein, signals derived from two types of reference materials are used. If signals were completely equally distributed, one-third of the time could be reduced. However, in reality, since many signals are present between two reference material signals, division of the searching space is carried out unequally. Taking into consideration this condition, reduction of approximately 45% is as it was expected, and it is considered that a sufficient effect can practically be achieved.

As stated above, it was found that the sample analyzing method of the present invention enables detection of signals whose amounts change in samples with a practically useful probability, and that useful measures are taken for the calculation method in the present invention.

Example 3

In Example 3, using tissue samples derived from clinical patients, protein-derived signals that significantly fluctuate among several pathologic groups were obtained. Thereafter, the MS/MS analysis was carried out using such signals, so as to identify several proteins thereof, thereby showing the effectiveness of the present method, in particular, the effectiveness thereof for the searching of a biomarker.

Specifically, adenocarcinoma in the lung was used as a target. Proteins were extracted from surgically excised tissues by a method described later, and then measured. The obtained profiles were divided, by pathologic diagnosis performed at a later date, into a group with metastasis to the lymph node and a group without such metastasis. Thereafter, signals that significantly fluctuated between both groups were picked up, and the thus obtained signals were subjected to the MS/MS analysis, so as to identify proteins.

Sample

Lung tissue sections that had surgically been excised from 36 individual patients with lung cancer were used as samples. These patients were classified by pathologic diagnosis into the total 4 groups using the following conditions: a group of patients with a large diameter of tumor; a group of patients with a small diameter of tumor; a group of patients with metastasis to the lymph node; and a group of patients without such metastasis.

More specifically, 10 patients were classified into a group with a small diameter of tumor and without metastasis to the lymph node. 11 patients were classified into the group with a large diameter of tumor and without metastasis to the lymph node. 12 patients were classified into a group with a small diameter of tumor and with metastasis to the lymph node. 3 patients were classified into a group with a large diameter of tumor and with metastasis to the lymph node.

Preparation of sample and protein fractionation

Each tissue section was crushed in a sample buffer solution used for sodium dodecyl sulfate (SDS)-polyacrylamide gel electrophoresis (PAGE). The composition of the sample buffer solution is as follows: 62.5 mM Tris-hydrochloric acid (pH 6.8), 2% w/v SDS, 5% v/v 2-mercaptoethanol, 10% v/v glycerin, and 0.0025% w/v bromophenol blue. This suspension was stirred at room temperature for 30 minutes, and then centrifuged to separate a supernatant from a precipitate. The concentration of a protein contained in the supernatant was measured by a modified Lowry method. To the sample supernatant corresponding to 100 $\mu$g of protein, a sample buffer solution with the same composition was added, resulting in the total 50 $\mu$l of a solution. AIM Tris aqueous solution was added to this solution, so that the pH was adjusted to pH 8.8. For reductive alkylation of a cysteine residue, 2 $\mu$l of 400 mM dithiothreitol was added thereto, and the mixture was incubated at 60°C for 30 minutes. Subsequently, 10 $\mu$l of a 400 mM iodoacetamide solution was added to the reaction solution, and the obtained mixture was left at room temperature under dark conditions for 60 minutes. Approximately 5 $\mu$l of 1.0 N hydrochloric acid was added, so that pH was returned to pH 6.8. This solution was subjected to Laemmli SDS-PAGE. Polyacrylamide gel used herein consisted of a discontinuous buffer solution system, namely, it consisted of concentrated gel (pH 6.8) in the upper portion and resolving gel (pH 8.8) in the lower portion. The concentrations of such polyacrylamide gel were 4% and 12.5%, respectively. The entire dimension of such gel consisted of a width of 14 cm, a height of 14 cm, and a thickness of 1 mm. A constant electric current of 10 mA was applied during the electrophoresis. When the electrophoretic front of pigment bromophenol reached a position at 48 mm on the resolving gel side from the interface between the concentrate gel and the resolving gel, the electrophoresis was terminated. The polyacrylamide gel was stirred in an aqueous solution containing 40% methanol and 10% acetic acid, so as to immobilize a protein separated in the polyacrylamide gel. Thereafter, the polyacrylamide gel was washed twice with water. For fractionation, the washed polyacrylamide gel was cut into 24 gel sections per sample. That is to say, the gel was cut into sections in a scalariform form at an equal width of 2 mm in the direction vertical to the electrophoretic direction. Thereafter, each section was further divided into cuboidal sections with a side of approximately 1 mm.

Preparation of standard protein

An internal standard protein that was immobilized in gel was added to each sample gel fraction. First, an aqueous egg-white lysozyme solution was mixed into an aqueous solution consisting of 12.5% acrylamide, 0.1% SDS, and 375 mM Tris-HCl (pH 8.8). Thereafter, *N,N,N',N'*-tetramethylethylenediamine and ammonium persulfate were further added thereto, so that acrylamide was polymerized in a portion with a width of 1 mm that was sandwiched between glass plates. A circle with a diameter of 1.5 mm was hollowed out of this gel containing an internal standard protein. The concentration of the protein in the aqueous solution before polymerization had previously been calculated, so that egg-white lysozyme with 2.5 pmol could be contained in a single gel section.

Digestion with protease

A single hollowed gel section containing a predetermined amount of standard protein was added to each sample gel fraction. Subsequently, the gel section in each fraction was washed with a sufficient amount of water, and then dehydrated with acetonitrile. Water and acetonitrile that remained in the gel section were distilled away under a reduced pressure, and an aqueous trypsin solution was then added to the gel section to such an extent that the gel section as a whole was immersed therein. The mixture was left on ice for 45 minutes. Thereafter, an aqueous solution that had not infiltrated into the gel was removed. A 50 mM aqueous ammonium bicarbonate solution was added thereto to such an extent that the gel section as a whole was immersed therein. The mixture was incubated at 37°C for 16 hours for a digestion reaction. Thereafter, peptide fragments contained in the gel section were extracted once with a 25 mM ammonium bicarbonate/50% acetonitrile aqueous solution, and then twice with a 5% formic acid/50% acetonitrile aqueous solution. The extracts were collected in a single container, followed by vacuum concentration.

LC-MS analysis

In order to obtain the three-dimensional profile of each peptide sample, the peptide sample was analyzed by the following operation using the following device (Kawakami T. et al., Jpn. J. Electrophoresis 44: 185-190 (2000)). First, the peptide sample that had been concentrated under a reduced pressure was dissolved in 45 $\mu$l of a solvent consisting of trifluoroacetic acid, acetonitrile, and water at a mixing ratio of 0.1 : 2 : 98. The obtained solution was defined as a dissolved solution.

Subsequently, using the autosampler PAL LC-1™ manufactured by CTC Analytics, 20 $\mu$l of the dissolved solution was introduced into the MAGIC MS™ C18 capillary column (inside diameter: 0.2 mm; length: 50 mm; grain diameter: 5 $\mu$m; pore size: 200 angstroms) manufactured by Michrom BioResources. The peptide was eluted using the MAGIC 2002™ HPLC system (Michrom BioResources). During this process, HPLC mobile phase A was a solvent produced by mixing formic acid, acetonitrile, and water at a volume ratio of 0.1 : 2 : 98. In contrast, the mixing ratio of components in mobile phase B was 0.1 : 90 : 10. The concentration of mobile phase B was increased from 5% to 85% in a linear gradient manner, so as to continuously elute peptide fragments. The flow rate was set at approximately I $\mu$l/min. The LC eluate was directly introduced into an ion source in the LCQ™ ion trap mass spectrometer (ThermoQuest) via the PicoChip™ needle (inside diameter: 20 $\mu$m) manufactured by New Objective. Fine adjustments were made for the distance of a NanoESI needle from a heating capillary. The spray voltage was not applied to the needle, but it was directly applied to the eluate. Gas was not used for spaying. The spray current was set at 3.0 mA.

Data processing

The total number of the obtained LC-MS profile data was 864 (36 samples x 24 bands). These profile data were converted into text files using Xcalibur™ utility software. Thereafter, the files were analyzed by the following procedures using programs written in the C language, the C++ language, and the Perl language.

(1) Signals with ionic intensity of $10^2$ or less were eliminated, so as to eliminate data with noise level.
(2) In order to reduce the processing time, m/z and retention time were quantized, so as to summarize data points. Specifically, in order that the retention time is set in increments of approximately 1, data points were searched in the neighborhood in the order of decreasing signal intensity with a time difference of 1 as the maximum limit, and data points in the range up to monotonic reduction were summarized as a signal. In addition, the m/z value in the original data was rounded off, such that the m/z was set in increments of 1. Data points having the same m/z value in the aforementioned time range were added together and accumulated.

(3) Signals derived from chicken egg-white lysozyme as a reference material were identified. That is to say, a data point with the highest ionic intensity was searched in a range before and after the m/z value and retention time value of the reference material that had been actually measured in a preliminary experiment. Subsequently, using the obtained data point as a center, data points, the ion intensity value of which was monotonously reduced and was greater than 0, were picked up around the above data point. All these data points were considered to be data points due to signals derived from the reference material. The total ionic intensity value of the signals derived from the reference material was considered to be the total sum of ionic intensity values of data points that were considered to be the signals derived from the reference material. Specifically, a signal with an m/z value derived from chicken egg-white lysozyme that was approximately 715, and a signal with an m/z value therefrom that was approximately 877, were used as reference signals. When these signals derived from the reference material were searched through the measurement data of samples, regarding m/z, searching was carried out within the range of the actual value ±1. Regarding the retention time, the signal with m/z of 715 was searched within the range of 10 minutes ±5, and the signal with m/z of 877 was searched within the range of 18 minutes ±5 minutes. However, when some signals were far different from other signals in terms of any one of the absolute intensity of the obtained signals derived from the reference material, the relative intensity in all the signals, and the intensity ratio between signals derived from two types of reference materials, the plot of each profile was individually confirmed, and adjustments were made such that the peak of a group of signals that were considered to be derived from the reference material came to the center of parameters during searching. Then, searching was carried out again. The ion intensity of each signal was divided by the total ion intensity value of the obtained reference material-derived signals, and the obtained value was multiplied by $10^7$, so that the signal intensity of the reference material-derived signals was corrected to be $10^7$. Moreover, for the sake of convenience, the retention time axis was subjected to linear transformation, so that the peak position of the signal with m/z of 715 and that of the signal with m/z of 877 became 10 minutes and 20 minutes, respectively, with regard to the retention time.

(4) With regard to profiles corresponding to 24 bands fractionated by SDS-PAGE, in order to guarantee the quantitative properties of a protein straddling the bands, a profile obtained by aligning all the bands was used as the profile of each sample. Specifically, using the profile alignment function of the sample analyzing program of the present invention, profiles of bands adjacent to each other were successively aligned, added together, and accumulated. That is to say, first, bands adjacent to each other were aligned, such that the aligned bands included a common band, such as 1+2, 2+3, 3+4, ... , 23+24 ..., and at the next stage, 1+2 and 2+3 were aligned so as to obtain 1~3. Thus, by aligning the bands such that at least one band was aligned, all the bands were finally aligned as a result of 6 stages of alignment operations, that is, by the final alignment of 1~17 with 9~24. The thus aligned band was divided by the number of aligning operations at the last stage, so as to allow the band to maintain quantitative properties.

The following parameters were used for alignment.

In the aforementioned formula (I), penalty $\alpha$ that depends on the difference (absolute value) on the time base was set at 1.0, penalty $\beta$ that depends on the difference on the signal intensity was set at 1.0 (wherein signal intensity was defined as an absolute value of the difference after conversion into a common logarithm), bonus point $\sigma$ for the correspondence of the points was set at 100, penalty $\pi$ for the non-correspondence of the points was set at 10, and bonus point $\theta(i,j) = S_m$ for the correspondence of the reference material-derived signals was set at 1,000. In addition, an output option regarding aligned profiles involves all the points including non-correspondence. Every time the alignment processing was terminated, the operation to summarize data points was carried out such that the retention time and m/z became resolution 1.0 and 1.0, respectively.

(5) In order to find proteins for distinguishing a group with metastasis to the lymph node from a group without such metastasis, in accordance with the aforementioned classification into 4 types of samples, profiles were first aligned in the group to obtain a summarized profile. Thereafter, such a profile alignment operation was carried out between the groups in the same manner. The parameters used in this alignment processing were the same as those used in the aforementioned alignment processing between the bands. Regarding the order of alignment, in the case of the alignment within the group, based on the evaluation function scores in the alignment processing under the same parameters, which had previously been carried out in a polyallel manner, profiles close to each other were successively aligned. In the case of the alignment between the groups, first, two groups with different tumor diameters were aligned in the group with metastasis to the lymph node, and two groups with different tumor diameters were aligned in the group without such metastasis were then aligned. Finally, the group with metastasis to the lymph node and the group without such metastasis were aligned.

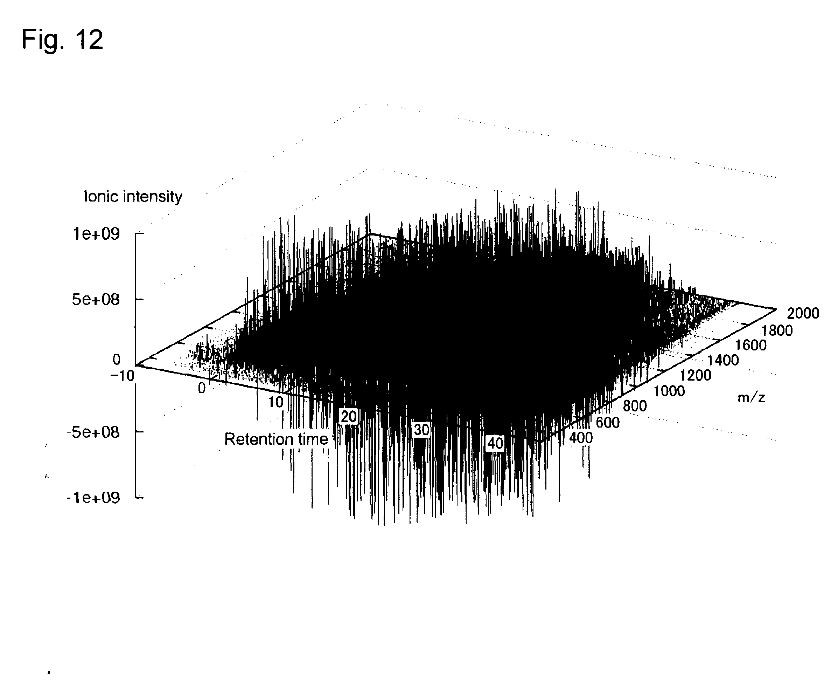

Figure 12 shows the finally aligned profile, wherein the signals existing in the positive group with metastasis to the lymph code are shown upward, and the signals existing in the negative group are shown in downward.

(6) The program was designed such that the routine was returned to the original data of 36 analytes x 24 bands as a starting point of alignment by reversely tracing the aforementioned alignment order. Thus, the program was designed such that all points on the profile obtained by finally aligning all the profiles correspond to the original data.

(7) With regard to each point on the finally summarized profile, each of the data derived from analytes with metastasis to the lymph node and the data derived from analytes without such metastasis were accumulated. Thereafter, the two-sided t-test was conducted on the difference in the mean values of both groups, thereby obtaining the p value based on the difference in the mean values of both groups and the above test.

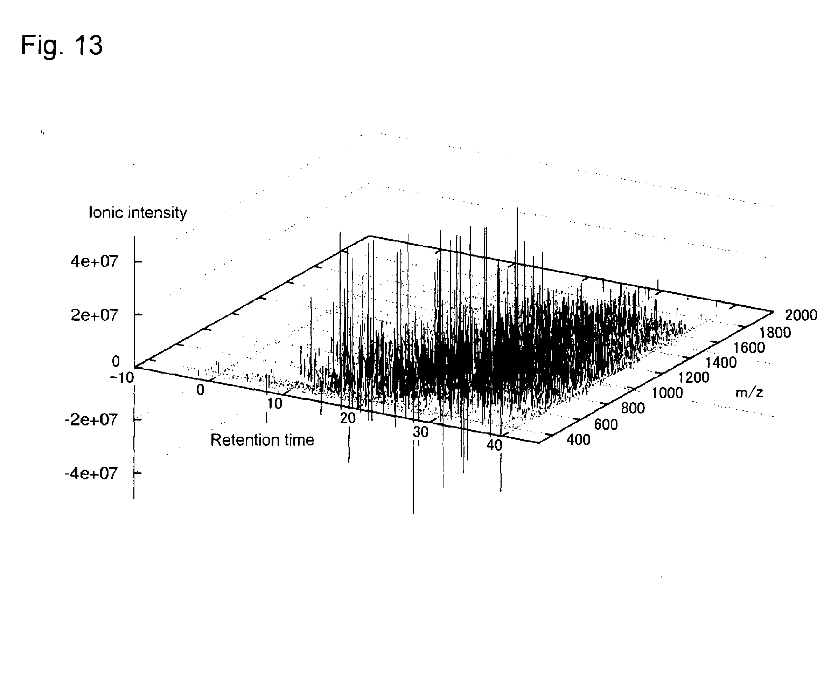

Figure 13 shows the points with a p value of less than 0.005 in the aforementioned test, in the same plot as used in Figure 12. At this stage, 5,889 signals were obtained.

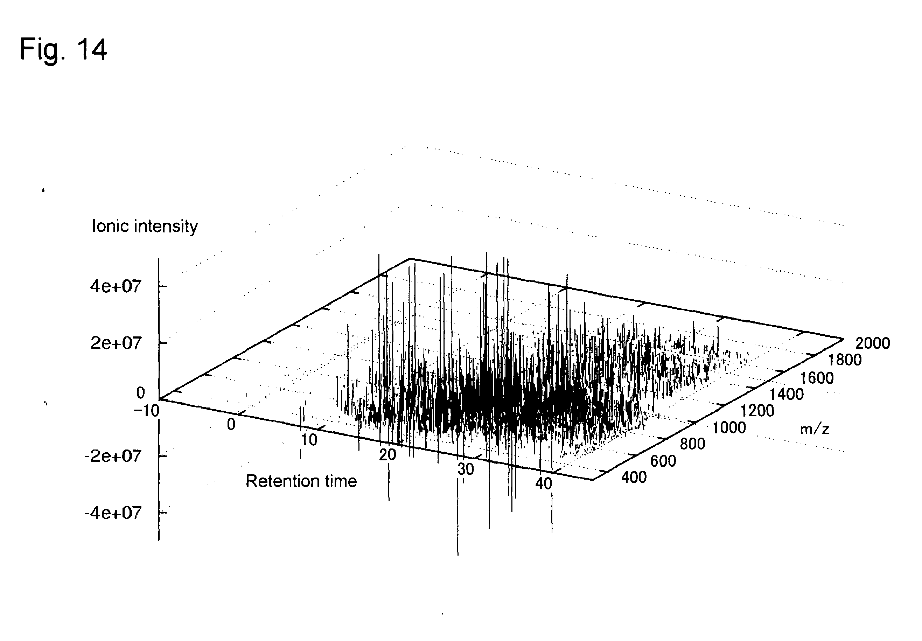

(8) Target MS/MS was carried out based on the information of the signals selected as described above, or the MS/MS analysis was carried out separately. Using protein identification software MASCOT™, proteins from which the signals were derived were identified. Figure 14 shows signals that were associated with the protein information by this identification. 2,753 signals (approximately a half) were associated with known proteins.

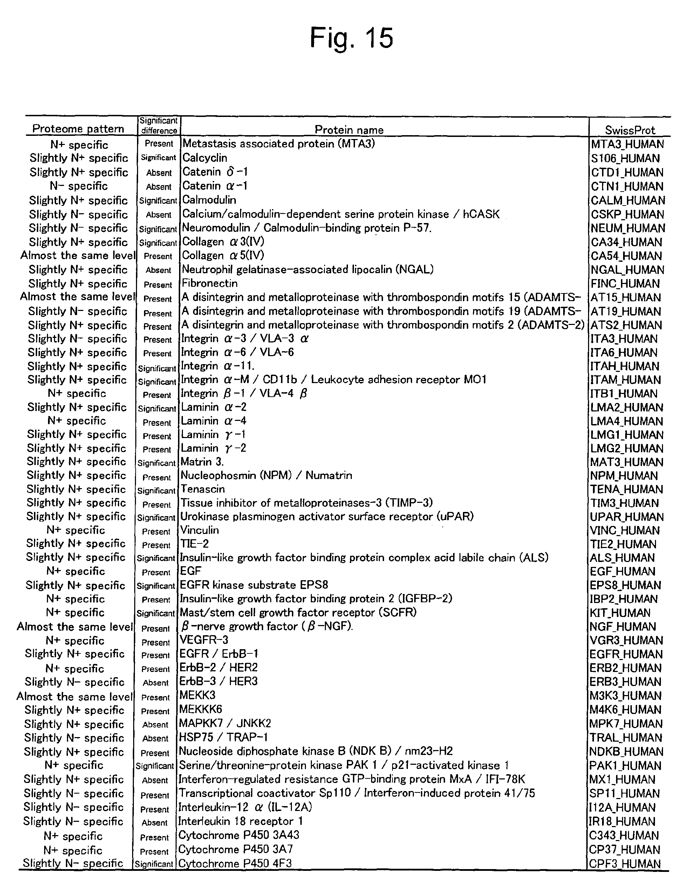

Finally, from among the aforementioned known proteins associated with the signals, several proteins that are reportedly involved in metastasis of cancers are shown in a list (Figure 15). Thus, it was revealed that proteins that are considered to be associated with metastasis of cancers can effectively be discovered by the system and program of the present invention.

As stated above, the sample analyzing system and program of the present invention are effective for analysis using actual clinical analytes, and in particular, enable detection of a pathological and/or clinical difference that is associated with the difference in the amount of protein. In addition, using the results, a protein is effectively identified. Accordingly, the sample analyzing system and program of the present invention are useful also for searching of a biomarker or the development of a new diagnostic method.

All publications, patents and patent applications cited herein are incorporated herein by reference in their entirety.

Industrial Applicability

As stated above in detail, the sample analyzing method and sample analyzing program of the present invention enable the analysis of components contained in a sample with excellent analysis ability. Accordingly, the present invention provides a sample analyzing method and a sample analyzing program that are extremely effective and useful when a large number of components contained in a sample as an analysis target are comprehensively analyzed.

In particular, the sample analyzing method and sample analyzing program of the present invention are significantly effective for the purpose of searching for a substance associated with the difference in pathologic conditions of a certain disease using actual clinical analytes. Thus, the present invention is extremely useful in that it can be used for a search for a biomarker or the development of a diagnostic method.

**Claims**

1. A sample analyzing method, which comprises:

   a step (a) of correcting at least a one-dimensional parameter in multi-dimensional data obtained as a result of the analysis of a sample; and
   a step (b) of comparing the corrected data obtained in said step (a) for multiple samples.

2. The sample analyzing method according to claim 1, wherein said multi-dimensional data is three-dimensional data consisting of a parameter indicating a mass-to-charge ratio, a parameter indicating ionic intensity, and a parameter indicating a retention time, obtained as a result of chromatography mass spectrometry, and wherein the parameter indicating a retention time is corrected in said step (a).

3. The sample analyzing method according to claim 1, wherein, in said step (a), profiles regarding parameters, from which a parameter as a correction target has been excluded, are used as reference profiles, and wherein using an evaluation function acting as a scale of position similarity regarding a plurality of reference profiles among multiple samples, the position of each profile is determined as a problem of finding an optimum solution which optimizes the value of said evaluation function.

4. The sample analyzing method according to claim 3, wherein said evaluation function is defined with one or more terms selected from the group consisting of the following terms (1) to (5):

   (1) a term regarding similarity and/or distance among profiles regarding a parameter of a correction target;
   (2) a term regarding similarity and/or distance among profiles regarding a reference profile;
   (3) a term regarding the degree of concordance of data points among profiles as comparison targets;
   (4) a term regarding the degree of discordance of data points among profiles as comparison targets;
   (5) a term regarding the degree of concordance or discordance of reference material-derived signals among profiles as comparison targets; and
   (6) a term regarding the degree of concordance in the previous comparison during repeated comparison operations.

5. The sample analyzing method according to claim 3, wherein, in said step (a), dynamic programming algorithm is used, when the value of said evaluation function is optimized as a problem of finding an optimum solution regarding said parameter of a correction target.

6. The sample analyzing method according to claim 5, wherein, in said dynamic programming algorithm, when the optimal correspondence of data points contained in a parameter of a correction target is evaluated by calculating scores, the score of a correspondence regarding data points derived from a reference material is set by a point-addition scoring system.

7. The sample analyzing method according to claim 5, wherein, in said dynamic programming algorithm, when the optimal correspondence of data points contained in a parameter of a correction target is evaluated by calculating scores, a constraint condition is set, in which a correspondence regarding data points derived from a reference material is necessarily corresponded at a designated point.

8. The sample analyzing method according to claim 1, wherein said sample comprises a protein group and/or a peptide group.

9. The sample analyzing method according to claim 1, wherein said multiple samples comprise a reference material.

10. The sample analyzing method according to claim 9, wherein said reference material is at least one type of peptide selected from the group consisting of peptide T (Ala-Ser-Thr-Thr-Asn-Tyr-Thr), $\beta$-casomorphin-7 (Tyr-Pro-Phe-Pro-Gly-Pro-Ile), and a structural analog thereof.

11. The sample analyzing method according to claim 9, wherein said reference material is added to said sample in a state where it is immobilized in gel.

12. A sample analyzing program for allowing a computer to execute:

   a procedure (a) of inputting multi-dimensional data obtained as a result of the analysis of a sample;
   a procedure (b) of correcting the data of at least a one-dimensional parameter from among the inputted multi-dimensional data; and
   a procedure (c) of comparing multi-dimensional data including the data corrected in said procedure (b) for multiple samples.

13. The sample analyzing program according to claim 12, wherein said multi-dimensional data is three-dimensional data consisting of a parameter indicating a mass-to-charge ratio, a parameter indicating ionic intensity, and a parameter indicating a retention time, obtained as a result of chromatography mass spectrometry, and wherein the parameter indicating a retention time is corrected in said procedure (b).

14. The sample analyzing program according to claim 12, wherein, in said procedure (b), profiles regarding parameters, from which a parameter as a correction target has been excluded, are used as reference profiles, and wherein using an evaluation function acting as a scale of position similarity regarding a plurality of reference profiles among multiple samples, the position of each profile is determined by optimizing the value of said evaluation function as a problem of finding an optimum solution.

15. The sample analyzing program according to claim 14, wherein said evaluation function is defined with one or more terms selected from the group consisting of the following terms (1) to (5):

   (1) a term regarding similarity and/or distance among profiles regarding a parameter of a correction target;
   (2) a term regarding similarity and/or distance among profiles regarding a reference profile;
   (3) a term regarding the degree of concordance of data points among profiles as comparison targets;
   (4) a term regarding the degree of discordance of data points among profiles as comparison targets;
   (5) a term regarding the degree of concordance or discordance of reference material-derived signals among profiles as comparison targets; and
   (6) a term regarding the degree of concordance in the previous comparison during repeated comparison operations.

16. The sample analyzing program according to claim 14, wherein, in said procedure (a), dynamic programming algorithm is used, when the value of said evaluation function is optimized as a problem of finding an optimum solution regarding said parameter of a correction target.

17. The sample analyzing program according to claim 16, wherein, in said dynamic programming algorithm, when the optimal correspondence of data points contained in a parameter of a correction target is evaluated by calculating scores, the score of a correspondence regarding data points derived from a reference material is set by a point-addition scoring system.

18. The sample analyzing program according to claim 16, wherein, in said dynamic programming algorithm, when the optimal correspondence of data points contained in a parameter of a correction target is evaluated by calculating scores, a constraint condition is set, in which a correspondence regarding data points derived from a reference material is necessarily corresponded at a designated point.

19. The sample analyzing program according to claim 12, wherein said sample comprises a protein group and/or a peptide group, and wherein multi-dimensional data derived from said protein group and/or peptide group are analyzed.

20. The sample analyzing program according to claim 12, wherein said multiple samples comprise reference materials, and wherein multi-dimensional data derived from these reference materials and multi-dimensional data derived from components contained in said samples are used in said procedure (b).

21. The sample analyzing program according to claim 20, wherein said reference material is at least one type of peptide selected from the group consisting of peptide T (Ala-Ser-Thr-Thr-Asn-Tyr-Thr), $\beta$-casomorphin-7 (Tyr-Pro-Phe-Pro-Gly-Pro-Ile), and a structural analog thereof.

22. The sample analyzing program according to claim 20, wherein said reference material is added to said sample in a state where it is immobilized in gel.

Fig. 1

Ionic intensity

1.6e+08
1.4e+08
1.2e+08
1e+08
8e+07
6e+07
4e+07
2e+07
0

Retention time

5  10  15  20  25  30  35  40  45

m/z

400  600  800  1000  1200  1400  1600  1800  2000

# Fig. 2

| m/z \ Retention time | 7 | 8 | 9 | 10 | 11 | 12 | 13 | 14 | 15 | 16 | 17 | 18 | 19 | 20 | 21 | 22 | 23 | 24 | .. |
|---|---|---|---|---|---|---|---|---|---|---|---|---|---|---|---|---|---|---|---|
| 553 | | | | | 223 | 121 | | | | | | | | | | | | | |
| 560 | | | | | | | | | | | 259 | 153 | | | | | | | |
| 563 | 564 | 882 | 96 | | | | | | | | | | | | | | | | |
| 590 | | | | | | | | 98 | 216 | | | | | | | | | | |
| 612 | | | | | | | | | | | | | | | 436 | 842 | 1291 | 461 | |
| 613 | | | | | | | | | 21 | | 332 | 48 | | | | | | | |
| : | | | | | | | | | | | | | | | | | | | |

Fig. 3

| Retention time / m/z | 5 | 6 | 7 | 8 | 9 | 10 | 11 | 12 | 13 | 14 | 15 | 16 | 17 | 18 | 19 | 20 | 21 | 22 | .. |
|---|---|---|---|---|---|---|---|---|---|---|---|---|---|---|---|---|---|---|---|
| 553 | | | | 321 | 212 | | | | | | | | | | | | | | |
| 560 | | | | | | | | | 92 | 323 | 112 | | | | | | | | |
| 563 | 481 | 769 | 112 | | | | | | | | | | | | | | | | |
| 590 | | | | | | | | 121 | 197 | | | | | | | | | | |
| 612 | | | | | | | | | 36 | 432 | 89 | | | | 332 | 692 | 952 | 215 | |
| 613 | | | | | | | | | | | | | | | | | | | |
| .. | | | | | | | | | | | | | | | | | | | |

Fig. 4

Fig. 5

Fig. 6

Fig. 7

Fig. 8

EP 1 626 274 A1

Fig. 9

EP 1 626 274 A1

Fig. 10

EP 1 626 274 A1

Fig. 11

Fig. 12

Fig. 13

Fig. 14

# Fig. 15

| Proteome pattern | Significant difference | Protein name | SwissProt |
|---|---|---|---|
| N+ specific | Present | Metastasis associated protein (MTA3) | MTA3_HUMAN |
| Slightly N+ specific | Significant | Calcyclin | S106_HUMAN |
| Slightly N+ specific | Absent | Catenin $\delta$-1 | CTD1_HUMAN |
| N- specific | Absent | Catenin $\alpha$-1 | CTN1_HUMAN |
| Slightly N+ specific | Significant | Calmodulin | CALM_HUMAN |
| Slightly N- specific | Absent | Calcium/calmodulin-dependent serine protein kinase / hCASK | CSKP_HUMAN |
| Slightly N- specific | Significant | Neuromodulin / Calmodulin-binding protein P-57. | NEUM_HUMAN |
| Slightly N+ specific | Significant | Collagen $\alpha$3(IV) | CA34_HUMAN |
| Almost the same level | Present | Collagen $\alpha$5(IV) | CA54_HUMAN |
| Slightly N+ specific | Absent | Neutrophil gelatinase-associated lipocalin (NGAL) | NGAL_HUMAN |
| Slightly N+ specific | Present | Fibronectin | FINC_HUMAN |
| Almost the same level | Present | A disintegrin and metalloproteinase with thrombospondin motifs 15 (ADAMTS- | AT15_HUMAN |
| Slightly N- specific | Present | A disintegrin and metalloproteinase with thrombospondin motifs 19 (ADAMTS- | AT19_HUMAN |
| Slightly N+ specific | Present | A disintegrin and metalloproteinase with thrombospondin motifs 2 (ADAMTS-2) | ATS2_HUMAN |
| Slightly N- specific | Present | Integrin $\alpha$-3 / VLA-3 $\alpha$ | ITA3_HUMAN |
| Slightly N+ specific | Present | Integrin $\alpha$-6 / VLA-6 | ITA6_HUMAN |
| Slightly N+ specific | Significant | Integrin $\alpha$-11. | ITAH_HUMAN |
| Slightly N+ specific | Significant | Integrin $\alpha$-M / CD11b / Leukocyte adhesion receptor MO1 | ITAM_HUMAN |
| N+ specific | Present | Integrin $\beta$-1 / VLA-4 $\beta$ | ITB1_HUMAN |
| Slightly N+ specific | Significant | Laminin $\alpha$-2 | LMA2_HUMAN |
| N+ specific | Present | Laminin $\alpha$-4 | LMA4_HUMAN |
| Slightly N+ specific | Present | Laminin $\gamma$-1 | LMG1_HUMAN |
| Slightly N+ specific | Present | Laminin $\gamma$-2 | LMG2_HUMAN |
| Slightly N+ specific | Significant | Matrin 3. | MAT3_HUMAN |
| Slightly N+ specific | Present | Nucleophosmin (NPM) / Numatrin | NPM_HUMAN |
| Slightly N+ specific | Significant | Tenascin | TENA_HUMAN |
| Slightly N+ specific | Present | Tissue inhibitor of metalloproteinases-3 (TIMP-3) | TIM3_HUMAN |
| Slightly N+ specific | Significant | Urokinase plasminogen activator surface receptor (uPAR) | UPAR_HUMAN |
| N+ specific | Present | Vinculin | VINC_HUMAN |
| Slightly N+ specific | Present | TIE-2 | TIE2_HUMAN |
| Slightly N+ specific | Significant | Insulin-like growth factor binding protein complex acid labile chain (ALS) | ALS_HUMAN |
| N+ specific | Present | EGF | EGF_HUMAN |
| Slightly N+ specific | Significant | EGFR kinase substrate EPS8 | EPS8_HUMAN |
| N+ specific | Present | Insulin-like growth factor binding protein 2 (IGFBP-2) | IBP2_HUMAN |
| N+ specific | Significant | Mast/stem cell growth factor receptor (SCFR) | KIT_HUMAN |
| Almost the same level | Present | $\beta$-nerve growth factor ($\beta$-NGF). | NGF_HUMAN |
| N+ specific | Present | VEGFR-3 | VGR3_HUMAN |
| Slightly N+ specific | Present | EGFR / ErbB-1 | EGFR_HUMAN |
| N+ specific | Present | ErbB-2 / HER2 | ERB2_HUMAN |
| Slightly N- specific | Absent | ErbB-3 / HER3 | ERB3_HUMAN |
| Almost the same level | Present | MEKK3 | M3K3_HUMAN |
| Slightly N+ specific | Present | MEKKK6 | M4K6_HUMAN |
| Slightly N+ specific | Absent | MAPKK7 / JNKK2 | MPK7_HUMAN |
| Slightly N- specific | Absent | HSP75 / TRAP-1 | TRAL_HUMAN |
| Slightly N+ specific | Present | Nucleoside diphosphate kinase B (NDK B) / nm23-H2 | NDKB_HUMAN |
| N+ specific | Significant | Serine/threonine-protein kinase PAK 1 / p21-activated kinase 1 | PAK1_HUMAN |
| Slightly N+ specific | Absent | Interferon-regulated resistance GTP-binding protein MxA / IFI-78K | MX1_HUMAN |
| Slightly N- specific | Present | Transcriptional coactivator Sp110 / Interferon-induced protein 41/75 | SP11_HUMAN |
| Slightly N- specific | Present | Interleukin-12 $\alpha$ (IL-12A) | I12A_HUMAN |
| Slightly N- specific | Absent | Interleukin 18 receptor 1 | IR18_HUMAN |
| N+ specific | Present | Cytochrome P450 3A43 | C343_HUMAN |
| N+ specific | Present | Cytochrome P450 3A7 | CP37_HUMAN |
| Slightly N- specific | Significant | Cytochrome P450 4F3 | CPF3_HUMAN |

**INTERNATIONAL SEARCH REPORT**

| International application No. |
| --- |
| PCT/JP2004/004621 |

| A. CLASSIFICATION OF SUBJECT MATTER |
| --- |
| Int.Cl$^7$  G01N27/62, G01N30/72 |

According to International Patent Classification (IPC) or to both national classification and IPC

| B. FIELDS SEARCHED |
| --- |
| Minimum documentation searched (classification system followed by classification symbols)<br>    Int.Cl$^7$  G01N27/62-27/70, G01N30/72 |

Documentation searched other than minimum documentation to the extent that such documents are included in the fields searched

| Jitsuyo Shinan Koho | 1922–1996 | Toroku Jitsuyo Shinan Koho | 1994–2004 |
| --- | --- | --- | --- |
| Kokai Jitsuyo Shinan Koho | 1971–2004 | Jitsuyo Shinan Toroku Koho | 1996–2004 |

Electronic data base consulted during the international search (name of data base and, where practicable, search terms used)
    JICST FILE(JOIS)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>Y | JP 7-198703 A  (Tokai Rubber Industries, Ltd.),<br>01 August, 1995 (01.08.95),<br>Full text; Figs. 1 to 12<br>(Family: none) | 1,12<br>2,13 |
| Y | J.D.Pinkston, et al., "Characterization of low molecular weight alkoxylated polymers using long column SFC/MS and an image analysis based quantitation approach", JOURNAL OF THE AMERICAN SOCIETY FOR MASS SPECTROMETRY Vol.13, No.10, October, 2002, pages 1195 to 1208 | 2,13 |
| A | JP 63-108260 A  (JEOL Ltd.),<br>13 May, 1988 (13.05.88),<br>Full text; Figs. 1 to 5<br>(Family: none) | 1-2,12-13 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    09 August, 2004 (09.08.04) | Date of mailing of the international search report<br>    24 August, 2004 (24.08.04) |
| --- | --- |
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2004/004621

C (Continuation). DOCUMENTS CONSIDERED TO BE RELEVANT

| Category* | Citation of document, with indication, where appropriate, of the relevant passages | Relevant to claim No. |
|---|---|---|
| A | JP 11-344482 A (JEOL Ltd.), 14 December, 1999 (14.12.99), Full text; Figs. 1 to 7 (Family: none) | 1-2,12-13 |

Form PCT/ISA/210 (continuation of second sheet) (January 2004)

**INTERNATIONAL SEARCH REPORT**

| International application No. |
| --- |
| PCT/JP2004/004621 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
      The technical feature common to claims 1, 2; 12, 13, claims 3-7, 14-18, claims 8, 19, and claims 9-11, 20-22 is that at least a one-dimensional parameter, out of multi-dimensional data collected by analyzing a sample, is corrected, and the corrected data is compared with data on other samples. However, such processing of analysis data is not novel since it is disclosed in documents such as JP 7-198703 A (Tokai Rubber Industries, Ltd.), 1 August, 1995 (01.08.95). Consequently, this common technical feature is not a special technical feature within the meaning of PCT Rule 13.2, second sentence, since it makes no contribution over the prior art.
      (Continued to extra sheet.)

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: 1, 2; 12, 13

**Remark on Protest**    ☐ The additional search fees were accompanied by the applicant's protest.

☐ No protest accompanied the payment of additional search fees.

Form PCT/ISA/210 (continuation of first sheet (2)) (January 2004)

EP 1 626 274 A1

| INTERNATIONAL SEARCH REPORT | International application No. |
|---|---|
| | PCT/JP2004/004621 |

Continuation of Box No.III of continuation of first sheet(2)

Since there exists no other common feature which can be considered as a special technical feature, no technical relationship within the meaning of PCT Rule 13 between the different inventions can be seen. Consequently, claims 1, 2; 12, 13, claims 3-7, 14-18, claims 8, 19, and claims 9-11, 20-22 do not satisfy the requirement of unity of invention.

Form PCT/ISA/210 (extra sheet) (January 2004)

46